# EUROPEAN PATENT APPLICATION

(11) **EP 1 201 268 A2**
(43) Date of publication of application: **02.05.2002**
(21) Application number: 01308953.7
(22) Date of filing: 22.10.2001
(51) Int. Cl.: A61P 25/00, A61K 45/06

(54) **Combinations of D4 dopamine receptor antagonists with acetylcholine esterase inhibitors**

(30) Priority: 26.10.2000 US 243543 P
(71) Applicant: Pfizer Products Inc., Groton, Connecticut 06340 (US)
(72) Inventor: Fliri, Anton Franz Josef, Pfizer Global Res. & Dev, Groton, Connecticut 06340 (US); Sanner, Mark Allen, Pfizer Global Res. and Dev., Groton, Connecticut 06340 (US); Zorn, Stevin Howard, Pfizer Global Res. and Dev., Groton, Connecticut 06340 (US)
(74) Representative: Ruddock, Keith Stephen

(57) **Abstract**

The present invention relates to a method of treating dementia or cognitive deficits associated with Alzheimer's Disease or Parkinson's Disease in a mammal, including a human, by administering to the mammal a D4 dopamine receptor antagonist in combination with an acetylcholine esterase inhibitor. It also relates to pharmaceutical compositions containing a pharmaceutically acceptable carrier, a D4 dopamine receptor antagonist and an acetylcholine esterase inhibitor.

## Description

### Background of the Invention

The present invention relates to a method of treating dementia or cognitive deficits associated with Alzheimer's Disease and Parkinson's Disease in a mammal, including a human, by administering to a mammal a D4 dopamine receptor antagonist in combination with an acetylcholine esterase inhibitor. It also relates to pharmaceutical compositions containing a pharmaceutically acceptable carrier, a D4 dopamine receptor antagonist and an acetylcholine esterase inhibitor.

Alzheimer's Disease (AD) is a degenerative brain disorder characterized clinically by progressive loss of memory, cognition, reasoning, judgment and emotional stability that gradually leads to profound mental deterioration and ultimately death. AD is a common cause of progressive mental failure (dementia) in aged humans and is believed to represent the fourth most common medical cause of death in the United States. AD has been observed in varied races and ethnic groups worldwide and presents a major present and future public health problem. The disease is currently estimated to affect about two to three million individuals in the United States alone. To date, AD has proven to be incurable and will increase worldwide as the human lifespan increases.

Alzheimer's Disease is associated with degeneration of cholinergic neurons in the basal forebrain that play a fundamental role in cognitive functions, including memory. Becker et al., Drug Development Research, 12, 163-195 (1988). As a result of such degeneration, patients suffering from the disease exhibit a marked reduction in acetylcholinesterase activity and choline uptake.

It is know that acetylcholine esterase inhibitors are effective in enhancing cholinergic activity and useful in improving the memory of Alzheimer's patients. By inhibiting the acetylcholinesterase enzyme, these compounds increase the level of the neurotransmitter acetylcholine, in the brain and thus enhance memory. Becker et al., supra, report that behavioral changes following cholinesterase inhibition appear to coincide with predicted peak levels of acetylcholine in the brain. They also discuss the efficacy of the three known acetylcholinesterase inhibitors pysostigmine, metrifonate, and tetrahydroaminoacridine.

U.S. Patent Nos. 5,750,542, 5,574,046 5,538,984, 5,965,575, and 6,124,321 all of which are assigned in common with the present application, also refer to heteroaryl amine acetylcholine esterase inhibitors and are incorporated by reference. United States Patent No. 4,895,481 is also incorporated by reference in its entirety.

Dopamine D4 receptors are more prevalent in the brains of schizophrenic patients (Seeman, et al. *Nature,* **1993,** *365,* 441) relative to normal controls. Dopamine receptor antagonists (especially the dopamine D4 receptor) are useful for the treatment of psychotic disorders, such as schizophrenia, and are accordingly of use in the treatment or prevention of psychotic disorders, especially affective psychosis, schizophrenia, and schizoaffective disorders.

WO 97/23482 (published July 3, 1997), WO 96/10571 (published April 11, 1996), WO 96/04250 (published February 15, 1996), WO 95/34555 (published December 21, 1995), WO 97/41108 (published November 6, 1997), WO 97/45419 (published December 4, 1997), WO 94/10162 (published May 11, 1994), and WO 94/10145 (published May 11, 1994) report that dopamine ligands are of use in the treatment and/or prevention of disorders of the dopamine system, including schizophrenia, nausea, Parkinson's disease, tardive dyskinesias and extrapyramidal side-effects associated with treatment by conventional neuroleptic agents, neuroleptic malignant syndrome, and disorders of hypothalamic-pituitary function such as hyperprolactinaemia and amenorrhoea. These patent applications are incorporated by reference in their entirety.

Dopamine is known to be a peripheral vasodilator; for example, it has been shown to exert a dilatory effect on the renal vascular bed. This implies that D4 dopamine receptors may be beneficial in controlling vascular blood flow.

### Summary of the Invention

The present invention relates to a pharmaceutical composition for the treatment of dementia or cognitive deficits associated with Alzheimer's Disease and Parkinson's Disease comprising: (a) a D4 dopamine receptor antagonist or a pharmaceutically acceptable salt thereof; (b) an acetylcholine esterase inhibitor or pharmaceutically acceptable salt thereof; and (c) a pharmaceutically acceptable carrier; wherein the active agents "a" and "b" above are present in amounts that render the composition effective in treating, respectively dementia or cognitive deficits associated with, Alzheimer's Disease and Parkinson's Disease.

This invention also relates to a method of treating dementia or cognitive deficits associated with Alzheimer's Disease and Parkinson's Disease in a mammal, comprising administering to said mammal, respectively, an Alzheimer's Disease and Parkinson's Disease dementia or cognitive deficits reducing effective amount of a pharmaceutical composition comprising: (a) an D4 dopamine receptor antagonist, or a pharmaceutically acceptable salt thereof; (b) a acetylcholine esterase inhibitor or pharmaceutically acceptable salt thereof; and (c) a pharmaceutically acceptable carrier; wherein the active agents "a" and "b" above are present in amounts that render the composition effective in treating dementia or cognitive deficits associated with Alzheimer's Disease and Parkinson's Disease.

This invention also relates to a method of treating dementia or cognitive deficits associated with Alzheimer's Disease and Parkinson's Disease in a mammal, comprising administering to said mammal: (a) a D4 dopamine receptor antagonist, or a pharmaceutically acceptable salt thereof; and (b) an acetylcholine esterase inhibitor or pharmaceutically acceptable salt thereof; wherein the active agents "a" and "b" above are administered in amounts that render the combination of the two agents effective in treating, respectively, dementia or cognitive deficits associated with Alzheimer's Disease and Parkinson's Disease.

It will be appreciated that when using a combination method of the present invention, referred to immediately above, both the D4 dopamine receptor antagonist and the acetylcholine esterase inhibitor will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the D4 dopamine receptor antagonist may be administered as a tablet and then, within a reasonable period of time, the acetylcholine esterase inhibitor may be administered either as an oral dosage form such as a tablet or a fast-dissolving oral dosage form. By a "fast dissolving oral formulation" is meant, an oral delivery form which when placed on the tongue of a patient, dissolves within about seconds.

The compositions of the present invention that contain a D4 dopamine receptor antagonist and an acetylcholine esterase inhibitor are useful for the treatment of dementia or cognitive deficits. As used herein, the term "dementia or cognitive deficits" includes disorientation impaired memory, judgement, intellect and a shallow labile affect.

The compositions of the present invention are especially useful for the treatment of dementia or cognitive deficits associated with Alzheimer's Disease and Parkinson's Disease. By the use of a combination of a D4 dopamine receptor antagonist and an acetylcholine esterase inhibitor in accordance with the present invention, it is possible to treat dementia or cognitive deficits associated with Alzheimer's Disease and Parkinson's Disease in patients for whom conventional dementia or cognitive deficit therapy might not be wholly successful. Examples of acetylcholine esterase inhibitors that may be used in the methods and pharmaceutical compositions of this invention are compounds of the formula I: 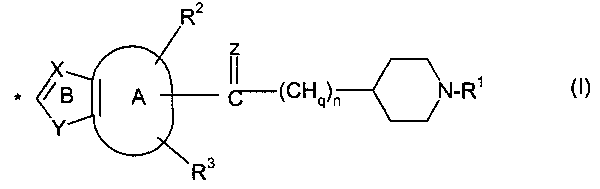
wherein one of R², R³ and the side chain containing 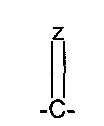 may optionally be attached to the carbon atom designated by an asterisk in ring B rather than to a member of ring A;
ring A is benzo, thieno, pyrido, pyrazino, pyrimido, furano, seleno, pyrrolo, thiazolo, or imidazolo;
R¹ is phenyl, phenyl-(C₁-C₆)alkyl, cinnamyl or heteroarylmethyl, wherein the heteroaryl moiety of said heteroarylmethyl is selected from imidazolo, thiazolo, thieno, pyrido and isoxazolo, and wherein said phenyl and said heteroaryl moiety may optionally be substituted with one or two substituents independently selected from (C₁-C₆)alkyl, (C₁-C₆)alkoxy and halo;
R² and R³ are independently selected from hydrogen, (C₁-C₆)alkoxy, (C₁-C₆)alkyl optionally substituted with from one to three fluorine atoms, benzyloxy, hydroxy, phenyl, benzyl, halo, nitro, cyano, COOR⁴, CONHR⁴, NR⁴R⁵, NR⁴COR⁵, or SOₚCH₂-phenyl wherein p is 0, 1 or 2;
or R² and R³ are attached to adjacent carbon atoms and form, together with the carbons to which they are attached, a five or six membered ring wherein each atom of the ring is carbon, nitrogen or oxygen (e.g., a methylenedioxy, ethylenedioxy or lactam ring);
R⁴ and R⁵ are independently selected from hydrogen and (C₁-C₆)alkyl, or R⁴ and R⁵, when part of said NR⁴R⁵, optionally form, together with the nitrogen to which they are attached, a ring containing four to eight members wherein one atom of the ring is nitrogen and the others are carbon, oxygen or nitrogen, or R⁴ and R⁵, when part of said NR⁴COR⁵, optionally form, together with the nitrogen and carbon to which they are attached, a four to eight membered lactam ring;
X is nitrogen or CH;
Y is oxygen, sulfur or NR⁶;
R⁶ is hydrogen, (C₁-C₆)alkyl, CO(C₁-C₆)alkyl or SO₂-phenyl, wherein the phenyl moiety of said SO₂-phenyl may optionally be substituted with from one to five substituents independently selected from (C₁-C₄) alkyl;
n is an integer from 1 to 4;
each q is independently 1 or 2; and
Z is oxygen or sulfur;
with the proviso that any CH_{q} group wherein q is 1 must be attached to one and only one other CH_{q} group wherein q is 1.

Preferred compounds are compounds of the formula I 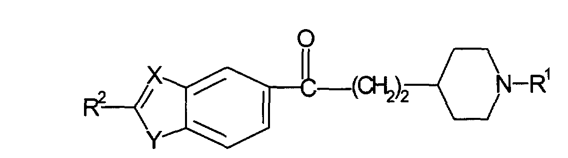 or 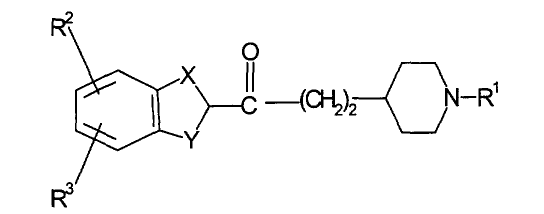 wherein X is CH, CCH₃, CCH₂CH₃ or N; Y is NH, NCH₃, NCH₂CH₃, S, O or NSO₂C₆H₅; R² and R³ are independently selected from the group consisting of (C₁-C₄)alkyl, chloro, fluoro, methoxy, amino and 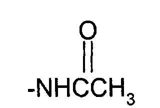 or R² and R³, together with the carbons to which they are attached, form a γ-lactam ring; and R¹ is benzyl, methoxybenzyl, fluorobenzyl or a group of the formula 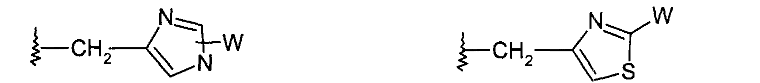 or 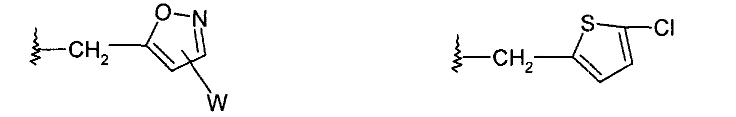 wherein W is hydrogen, (C₁-C₆)alkyl, phenyl or benzyl.

Specific preferred compounds of formula I are:
1-(2-methyl-1H-benzimidazol-5-yl)-3-[1-(phenylmethyl)-4-piperidinyl]-1-propanone;
1-(2-phenyl-1 H-benzimidazol-5-yl)-3-[1-(phenylmethyl)-4-piperidinyl]-1-propanone;
1-(1-ethyl-2-methyl-1H-benzimidazol-5-yl)-3-[1 -(phenylmethyl)-4-piperidinyl]-1-propanone;
1 -(2-methyl-6-benzothiazolyl)-3-[1 -(phenylmethyl)-4-piperidinyl]-1 -propanone;
1 -(2-methyl-6-benzothiazolyl)-3-[1-[(2-methyl-4-thiazolyl)methyl]-4-piperidinyl]-1-propanone;
1-(5-methyl-benzo[b]thien-2-yl)-3-[1-(phenylmethyl)-4-piperidinyl]-1-propanone;
1-(6-methyl-benzo[b]thien-2-yl)-3-[1-(phenylmethyl)-4-piperidinyl]-1-propanone;
1-(3,5-dimethyl-benzo[b]thien-2-yl)-3-[1 -(phenylmethyl)-4-piperidinyl]-1 -propanone;
1-(benzo[b]thien-2-yl)-3-[1 -(phenylmethyl)-4-piperidinyl]-1 -propanone;
1-(benzofuran-2-yl)-3-[1 -(phenylmethyl)-4-piperidinyl]-1 -propanone;
1-(1-phenylsulfonyl-6-methyl-indol-2-yl)-3-[1 -(phenylmethyl)-4-piperidinyl]-1-propanone;
1 -(6-methyl-indol-2-yl)-3-[1-(phenylmethyl)-4-piperidinyl]-1 -propanone;
1-(1-phenylsulfonyl-5-amino-indol-2-yl)-3-[1 -(phenylmethyl)-4-piperidinyl]-1 -propanone;
1-(5-amino-indol-2-yl)-3-[1-(phenylmethyl)-4-piperidinyl]-1-propanone; and
1-(5-acetylamino-indol-2-yl)-3-[1 -(phenylmethyl)-4-piperidinyl]-1-propanone.

Examples of other compounds of the invention are:
1 -(6-quinolyl)-3-[1 -(phenylmethyl)-4-piperidinyl]-1 -propanone;
1-(5-indolyl)-3-[1-(phenylmethyl)-4-piperidinyl]-1 -propanone;
1 -(5-benzthienyl)-3-[1-(phenylmethyl)-4-piperidinyl]-1 -propanone;
1 -(6-quinazolyl)-3-[1-(phenylmethyl)-4-piperidinyl]-1 -propanone;
1-(6-benzoxazolyl)-3-[1-(phenylmethyl)-4-piperidinyl]-1-propanone;
1 -(5-benzofuranyl)-3-[1-(phenylmethyl)-4-piperidinyl]-1-propanone;
1 -(5-methyl-benzimidazol-2-yl)-3-[1 -(phenylmethyl)-4-piperidinyl]-1 -propanone;
1 -(6-methyl-benzimidazol-2-yl)-3-[1-(phenylmethyl)-4-piperidinyl]-1 -propanone;
1 -(5-chloro-benzo[b]thien-2-yl)-3-[1-(phenylmethyl)-4-piperidinyl]-1-propanone;
1 -(5-azaindol-2-yl)-3-[1 -(phenylmethyl)-4-piperidinyl]-1 -propanone;
1 -(6-azabenzo[b]thien-2-yl)-3-[1 -(phenylmethyl)-4-piperidinyl]-1 -propanone;
1-(1H-2-oxo-pyrrolo[2N,3N,5,6]benzo[b]thieno-2-yl)-3-[1-(phenylmethyl)-4-piperidinyl]-1-propanone;
1-(6-methyl-benzothiazol-2-yl)-3-[1 -(phenylmethyl)-4-piperidinyl]-1 -propanone;
1 -(6-methoxy-indol-2-yl)-3-[1 -(phenylmethyl)-4-piperidinyl]-1 -propanone;
1 -(6-methoxy-benzo[b]thien-2-yl)-3-[1 -(phenylmethyl)-4-piperidinyl]-1 -propanone;
1-(6-acetylamino-benzo[b]thien-2-yl)-3-[1-(phenylmethyl)-4-piperidinyl]-1-propanone; and
1 -(5-acetylamino-benzo[b]thien-2-yl)-3-[1 -(phenylmethyl)-4-piperidinyl]-1 -propanone.

The compounds of formula I may have optical centers and may therefore occur in different isomeric forms. The invention includes all isomers of such compounds having formula I, including mixtures thereof.

Another example of an acetylcholine esterase inhibitors that can be used in the method and pharmaceutical composition of this invention are compound of the formula II 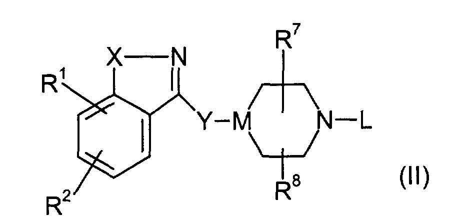 wherein R¹ and R² are independently selected from hydrogen, (C₁-C₆)alkoxy, benzyloxy, phenoxy, hydroxy, phenyl, benzyl, halo, nitro, cyano, -COOR⁵, -CONHR⁵, -NR⁵R⁶, -NR⁵COR⁶, -OCONR⁵R⁶, -NHCOOR⁵, (C₁-C₆)alkyl optionally substituted with from 1 to 3 fluorine atoms; SOₚCH₂-phenyl or SOₚ(C₁-C₆)alkyl, wherein p is 0, 1 or 2; pyridylmethyloxy or thienylmethyloxy; wherein the phenyl moieties of said phenoxy, benzyloxy, phenyl and benzyl groups, and the pyridyl and thienyl moieties of said pyridylmethyloxy and thienylmethyloxy may optionally be substituted with 1 or 2 substituents independently selected from halo, (C₁-C₄)alkyl, trifluoromethyl, (C₁-C₄)alkoxy, cyano, nitro and hydroxy; 2-oxazolyl, 2-thiazolyl and benzenesulfonamide;
or R¹ and R², when attached to adjacent carbon atoms and when X is oxygen or sulfur may form, together with the carbon atoms to which they are attached, a group of the formula 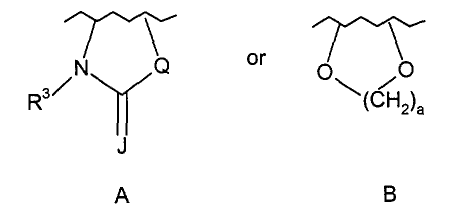
wherein J is oxygen, sulfur or NR⁴ wherein R⁴ is hydrogen or (C₁-C₄)alkyl, "a" is 1 or 2, R³ is hydrogen or (C₁-C₄)alkyl and Q is oxygen, sulfur, NH, CHCH₃, C(CH₃)₂, -CH=CH-, or (CH₂)_{I} wherein I is an integer from 1 to 3;
X is oxygen, sulfur, -CH=CH-, -CH=N-, -N=CH-, -N=N-, or NR⁴ wherein R⁴ is hydrogen or (C₁-C₄) alkyl;
Y is -(CH₂)ₘ-, -CH=CH(CH₂)ₙ-, -NR⁴(CH₂)ₘ-, or -O(CH₂)ₘ-
wherein R⁴ is defined as above, n is an integer from 0 to 3 and m is an integer from 1 to 3;
R⁵ and R⁶ are each independently selected from hydrogen, (C₁-C₆)alkyl, phenyl or benzyl, wherein the phenyl moieties of said phenyl and benzyl may optionally be substituted with 1 or 2 substituents independently selected from fluoro, chloro, bromo, iodo, (C₁-C₄) alkyl, trifluoromethyl, (C₁-C₄) alkoxy, cyano, nitro and hydroxy, or NR⁵R⁶ together form a 4 to 8 membered ring wherein one atom of the ring is nitrogen and the others are carbon, oxygen or nitrogen (e.g. pyrrolidinyl, piperidinyl, morpholino, piperazinyl or N-methylpiperazinyl), or NR⁵COR⁶ together form a 4 to 8 membered cyclic lactam ring;
M is -CH- or nitrogen;
L is phenyl, phenyl-(C₁-C₆)alkyl, cinnamyl or pyridylmethyl, wherein the phenyl moieties of said phenyl and phenyl-(C₁-C₆)alkyl may optionally be substituted with 1 to 3 substituents independently selected from (C₁-C₆)alkyl, (C₁-C₆)alkoxy, (C₁-C₄)alkoxycarbonyl, (C₁-C₄)alkylcarbonyl or halo; or L is a group of the formula 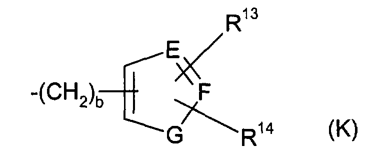 wherein b is an integer from 1 to 4, R¹³ and R¹⁴ are independently selected from hydrogen, (C₁-C₄) alkyl, halo and phenyl, E and F are independently selected from -CH- and nitrogen, and G is oxygen, sulfur or NR⁴ wherein R⁴ is defined as above, with the proviso that when E and F are both nitrogen, one of R¹³ and R¹⁴ is absent; and
R⁷ and R⁸ are independently selected from hydrogen, (C₁-C₆)alkyl, (C₁-C₆)alkoxycarbonyl, (C₁-C₆)alkylcarbonyl and (C₁-C₆)alkoxy, with the proviso that said (C₁-C₆)alkoxy is not attached to a carbon that is adjacent to a nitrogen.

This invention also relates to the pharmaceutically acceptable acid addition salts of compounds of the formula II. Examples of such pharmaceutically acceptable acid addition salts are the salts of hydrochloric acid, p-toluenesulfonic acid, maleic acid, fumaric acid, citric acid, succinic acid, salicylic acid, oxalic acid, hydrobromic acid, phosphoric acid, methanesulfonic acid, tartaric acid, di-p-toluoyl tartaric acid, and mandelic acid.

The term "halo", as used herein, includes chloro, bromo, iodo or fluoro.

The term "(C₁-C₄) alkylcarbonyl", as used herein, refers to a substituent of the formula 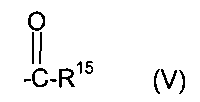 wherein R¹⁵ is (C₁-C₄) alkyl.

The term "(C₁-C₄) alkoxycarbonyl", as used herein, refers to a substituent of the formula V above, wherein R¹⁵ is (C₁-C₄) alkoxy.

The term "(C₁-C₆) alkoxycarbonyl", as used herein, refers to a substituent of the formula V above, wherein R¹⁵ is (C₁-C₆) alkoxy.

The term "(C₁-C₆)alkylcarbonyl", as used herein, refers to a substituent of the formula (V) above, wherein R¹⁵ is (C₁-C₆)alkyl.

Preferred compounds are compounds of the formula II wherein X is oxygen or sulfur, Y is -CH₂-, -CH₂-CH₂-, M is -CH- and L is benzyl, and the pharmaceutically acceptable salts of such compounds.

Specific preferred compounds of this invention are:
6-hydroxy-3-[2-[1-(phenylmethyl)-4-piperidinyl]ethyl]-1,2-benzisoxazole;
5-methyl-3-[2-[1-(phenylmethyl)-4-piperidinyl]ethyl]-1,2-benzisoxazole;
6-methoxy-3[2-[-1(phenylmethyl)-4-piperidinyl]ethyl]-1,2-benzisoxazole;
6-acetamido-3-[2-[1-(phenylmethyl)-4-piperidinyl]-ethyl]-1,2-benzisoxazole;
6-amino-3-[2-[1-(phenylmethyl)-4-piperidinyl]ethyl]-1,2-benzisoxazole;
6-(4-morpholinyl)-3-[2-[1-(phenylmethyl)-4-piperidinyl]ethyl]-1,2-benzisoxazole;
5,7-dihydro-3-[2-[1-(phenylmethyl)-4-piperidinyl]ethyl]-6H-pyrrolo[4,5-f]-1,2-benzisoxazol-6-one;
3-[2-[1-(phenylmethyl)-4-piperidinyl]ethyl]-1,2-benzisothiazole;
and the pharmaceutically acceptable salts of such compounds.

Examples of other compounds of the formula I are:
3-[2-[1 -(phenylmethyl)-4-piperidinyl]ethenyl]-1,2-benzisoxazole;
6-phenylamino-3-[2-[1-(phenylmethyl)-4-piperidinyl]ethyl]-1,2,-benzisoxazole;
6-(2-thiazoly)-3-[2-[1-(phenylmethyl)-4-piperidinyl]ethyl]-1,2-benzisoxazole;
6-(2-oxazolyl)-3-[2-[1-(phenylmethyl)-4-piperidinyl]ethyl]-1,2-benzisoxazole;
6-pyrrolidinyl-3-[2-[1-(phenylmethyl)-4-piperidinyl]ethyl]-1,2-benzisoxazole;
5,7-dihydro-5,5-dimethyl-3-[2-[1-(phenylmethyl)-4-piperidinyl]ethyl]-6H-pyrrolo[4,5-f]-1,2-benzisoxazole-6-one;
6,8-dihydro-3-[2-[1-(phenylmethyl)-4-piperidinyl]ethyl]-7H-pyrrolo[5,4-g]-1,2-benzisoxazole-7-one; and
3-[2-[1-(phenylmethyl)-4-piperidinyl]ethyl]-5,6,8-trihydro-7H-isoxazolo[4,5-g]-quinolin-7-one.

The compounds of formula II may have optical centers and may therefore occur in different isomeric forms. The invention includes all stereoisomers of such compounds of formula II, including mixtures thereof.

Another example of acetylcholine esterase inhibitors that can be used in the methods and pharmaceutical compositions of this invention are compounds of the formula III 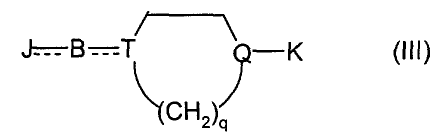
in which J is
   (a) a group, substituted or unsubstituted, selected from the group consisting of (1) phenyl, (2) pyridyl, (3) pyrazyl, (4) quinolyl, (5) cyclohexyl, (6) quinoxalyl and (7) furyl;
   (b) a monovalent or divalent group, in which the phenyl may have a substituent(s), selected from the group consisting of (1) indanyl, (2) indanonyl, (3) indenyl, (4) indenonyl, (5) indanedionyl, (6) tetralonyl, (7) benzosuberonyl, (8) indanolyl and (9) C₆ H₅ -CO-CH(CH₃)-;
   (c) a monovalent group derived from a cyclic amide compound;
   (d) a lower alkyl or
   (e) a group of R²¹ -CH=CH- in which R²¹ is hydrogen or a lower alkoxycarbonyl;
B is -(CHR²²)ᵣ -, -CO-(CHR²²)ᵣ -, -NR⁴ -(CHR²²)ᵣ -, R⁴ being hydrogen, a lower alkyl, an acyl, a lower alkylsulfonyl, phenyl, a substituted phenyl, benzyl or a substituted benzyl, -CO-NR⁵-(CHR²²)ᵣ -, R⁵ being hydrogen, a lower alkyl or phenyl, -CH=CH-(CHR²²)ᵣ -, -OCOO-(CHR²²)ᵣ-, -OOC-NH-(CHR²²)ᵣ-, -NH-CO-(CHR²²)ᵣ-, -CH₂-CO-NH-(CHR²²)ᵣ -, -(CH₂)₂ -NH-(CHR²²)ᵣ -, -CH(OH)-(CHR²²)ᵣ-, r being zero or an integer of 1 to 10, R²² being hydrogen or methyl so that one alkylene group may have no methyl branch or one or more methyl branch, =(CH-CH=CH)_{b}-, b being an integer of 1 to 3, =CH-(CH₂)_{c} -, c being zero or an integer of 1 to 9, (CH-CH)_{d}=, d being zero or an integer of 1 to 5; -CO-CH=CH-CH₂ -, -CO-CH₂ -CH(OH)-CH₂-, -CH(CH₃)-CO-NH-CH₂-, -CH=CH-CO-NH-(CH₂)₂-, -NH-, -O-, -S-, a dialkylaminoalkylcarbonyl or a lower alkoxycarbonyl;
T is a nitrogen or carbon;
Q is nitrogen, carbon or 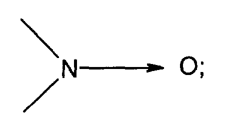
q is an integer of 1 to 3;
K is hydrogen, phenyl, a substituted phenyl, an arylalkyl in which the phenyl may have a substituent, cinnamyl, a lower alkyl, pyridylmethyl, a cycloalkylalkyl, adamantanemethyl, furylmenthyl, a cycloalkyl, a lower alkoxycarbonyl or an acyl; and shows a single bond or a double bond.

In the compounds having the formula (III), it is preferable that J is (a) or (b). In the definition (b), monovalent groups of (2), (3) and (5) and divalent groups of (2) are preferable. In the definition of B, -(CHR22) r-, =(CH-CH=CH)b-, =CH-(CH2)c- and =(CH-CH)d= are preferable. These preferable groups of (B) may be connected with (b) ofJ, in particular (2) of (b).

It is preferable in the formula III that Q is nitrogen, T is carbon and q is 1 or 3; and Q is carbon, T is nitrogen and q is 2.

It is most preferable that Q is nitrogen, T is carbon and q is 2.

It is preferable that K is a phenylalkyl or a phenylalkyl having a substituent(s) on the phenyl.

Preferable compounds of the invention include:
1-benzyl-4-((5,6-dimethoxy-1 -indanon)-2-yl)methylpiperidine,
1-benzyl-4-((5,6-dimethoxy-1-indanon)-2-ylidenyl)methylpiperidine,
1 -benzyl-4-((5-methoxy-1 -indanon)-2-yl)methylpiperidine,
1-benzyl-4-((5,6-diethoxy-1-indanon)-2-yl)methylpiperidine,
1-benzyl-4-((5,6-methnylenedioxy-1-indanon)-2-yl)methylpiperidine,
1-(m-nitrobenzyl)-4-((5,6-dimethoxy-1-indanon)-2-yl)methylpiperidine,
1-cyclohexymethyl-4-((5,6-dimethoxy-1-indanon)-2-yl)methylpiperidine,
1-(m-florobenzyl)-4-((5,6-dimethoxy-1-indanon)-2-yl)methylpiperidine,
1 -benzyl-4-((5,6-dimethoxy-1 -indanon)-2-yl)propylpiperidine,
1-benzyl-4-((5-isopropoxy-6-methoxy-1-indanon)-2-yl)methylpiperidine and
1-benzyl-4-((5,6-dimethoxy-1-indanolidenyl)-2-yl)propenylpiperidine, having the below shown formula,

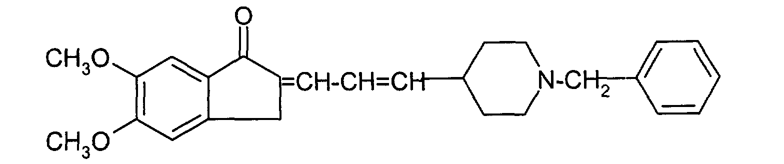

The preferable compound has the above shown formula in which J is (b). The group (b) includes ten groups having the respective formulae shown below. S is hydrogen or a substituent such as a lower alkyl having 1 to 6 carbon atoms and a lower alkoxy having 1 to 6 carbon atoms. Among the substituents, methoxy is most preferable. t is an integer of 1 to 4. The phenyl is most preferred to have 1 to 3 methoxy groups thereon. (S)ₜ may form methylene dioxy group or ethylene dioxy group on two adjacent carbon atoms of the phenyl group.

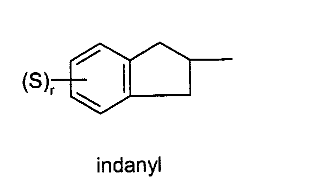

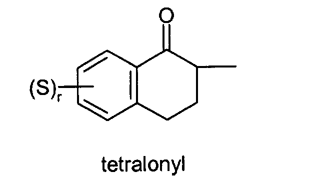

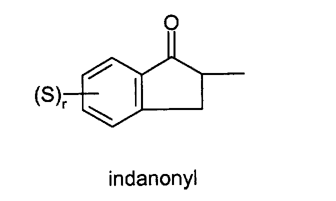

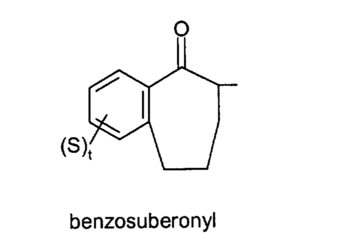

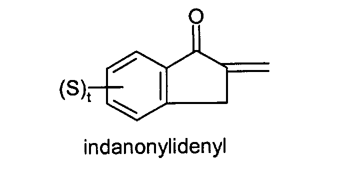

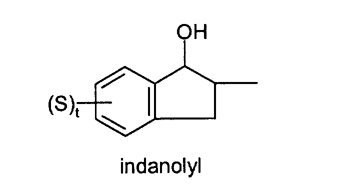

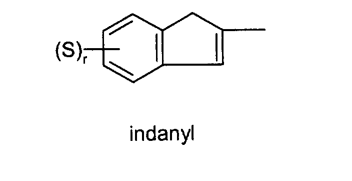

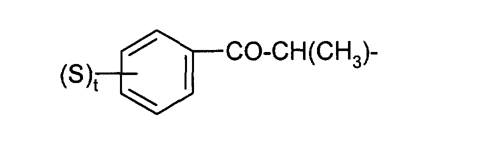

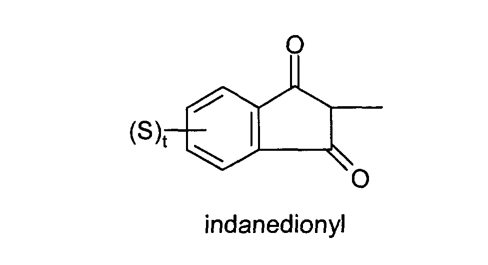

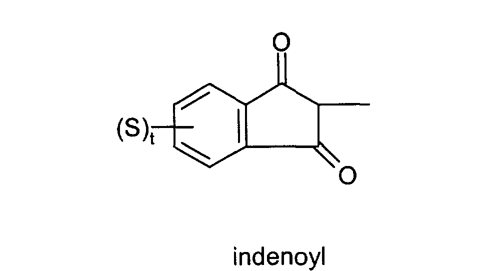

A preferable definition of B includes -(CHR²²)ᵣ-, -CO-(CHR²²)ᵣ -, =(CH―CH=CH)_{b}-,=CH-(CH₂)_{c}- and =(CH-CH)_{d}=. The group of -(CHR²²)ᵣ - in which R²² is hydrogen and r is an integer of 1 to 3 and then the group of =CH-(CH₂)_{c} -are most preferable.

In the above defined cyclic amine compound of the invention, it is preferable that J in the formula is (b) the monovalent or divalent group. In the definition (b), indanonyl, indanedionyl and indenyl are most preferable, optionally having a substituent(s) on the phenyl.

In the definition B, -(CHR²²)ᵣ- and =CH-(CH₂)_{c} - are preferable.

In the ring including T and Q, it may be a 5-, 6-or 7-membered ring. It is preferable that Q is nitrogen, T is carbon or nitrogen and n is 2; Q is nitrogen, T is carbon and n is 1 or 3; and Q is carbon, T is nitrogen and n is 2.

In the definition K, phenyl, an arylalkyl and cinnamyl are preferable, optionally having a substituent(s) on the phenyl.

Examples of D4 dopamine receptor antagonists that may be used in the methods and pharmaceutical compositions of this invention are compounds of the formula IV: 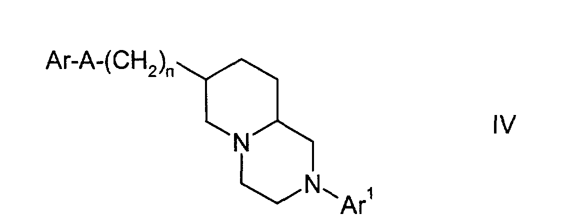
wherein Ar is phenyl, naphthyl, benzoxazolonyl, indolyl, indolonyl, benzimidazolyl, quinolyl, furyl, benzofuryl, thienyl, benzothienyl, oxazolyl, or benzoxazolyl;
Ar¹ is phenyl, pyridinyl, pyridazinyl, pyrimidinyl, or pyrazinyl;
A is O, S, SO, SO₂, C=O, CHOH, or -(CR³R⁴)-;
n is 0, 1 or 2;
each of Ar and Ar¹ may be independently and optionally substituted with one to four substituents independently selected from the group consisting of fluoro, chloro, bromo, iodo, cyano, nitro, thiocyano, -SR, -SOR, -SO₂R, -NHSO₂R, -(C₁-C₆)alkoxy, -NR¹R², -NRCOR¹, -CONR¹R², Ph, -COR, COOR, -(C₁-C₆)alkyl, -(C₁-C₆)alkyl substituted with one to six halogens, -(C₃-C₆)cycloalkyl, and trifluoromethoxy;
each and every R, R¹, and R² is independently selected from the group consisting of hydrogen, -(C₁-C₆)alkyl, -(C₁-C₆)alkyl substituted with one to thirteen halogens selected from fluorine, chlorine, bromine and iodine, phenyl, benzyl, -(C₂-C₆)alkenyl, -(C₃-C₆)cycloalkyl, and -(C₁-C₆)alkoxy;
each and every R³ and R⁴ is independently selected from the group consisting of hydrogen, methyl, ethyl, n-propyl, or i-propyl;
diastereomeric and optical isomers thereof; and
pharmaceutically acceptable salts thereof.

In another aspect, this invention relates to compounds of formula IV wherein
Ar is phenyl, naphthyl, benzoxazolonyl, indolyl, indolonyl, benzimidazolyl, or quinolyl;
A is O, S, SO₂, C=O, CHOH, or CH₂;
n is 0 or 1,
wherein Ar and Ar¹ may be independently substituted with up to three substituents independently selected from the group consisting of fluoro, chloro, cyano, -NR¹R², -(C₁-C₆)alkoxy, -COOR, -CONR¹R², and -(C₁-C₆)alkyl and the pharmaceutically acceptable salts thereof.

In addition compounds of formula IV wherein
A is O or S;
n is 1;
Ar is phenyl or substituted phenyl, and the pharmaceutically acceptable salts thereof.

In addition, compounds of formula IV wherein
A is CH₂;
n is 0;
Ar is benzoxazolonyl or substituted benzoxazolonyl; and the pharmaceutically acceptable salts thereof.

In addition, compounds of formula IV wherein
A is CH₂;
n is 0;
Ar is indolyl or substituted indolyl; and the pharmaceutically acceptable salts thereof.

In another aspect, this invention relates to compounds of formula I wherein
A is C=O or CHOH;
n is 0 or 1;
Ar is phenyl or substituted phenyl; and the pharmaceutically acceptable salts thereof.

In addition, compounds of formula IV wherein
A is O;
Ar is fluorophenyl, difluorophenyl or cyanophenyl;
Ar¹ is chloropyridinyl; and the pharmaceutically acceptable salts thereof.

In addition, compounds of formula IV wherein
A is O;
Ar is fluorophenyl, difluorophenyl or cyanophenyl;
Ar¹ is fluoropyrimidinyl; and the pharmaceutically acceptable salts thereof.

In addition, compounds of formula IV wherein
A is O;
Ar is fluorophenyl, difluorophenyl or cyanophenyl;
Ar¹ is fluorophenyl; and the pharmaceutically acceptable salts thereof.

In addition, compounds of formula IV wherein
Ar1 is 5-chloro-pyridin-2-yl; and the pharmaceutically acceptable salts thereof.

In addition, compounds of formula IV wherein
Ar¹ is 5-fluoro-pyrimidin-2-yl; and the pharmaceutically acceptable salts thereof.

In a preferred aspect of the invention, A is O.

In another aspect A is S, SO, or SO₂.

In another aspect A is C=O or CHOH.

In another preferred aspect A is CH₂.

In another preferred aspect Ar is phenyl or substituted phenyl.

In another preferred aspect Ar is naphthyl or substituted naphthyl.

In another preferred aspect Ar is benzoxazolonyl or substituted benzoxazolonyl.

In another preferred aspect Ar is indolyl or substituted indolyl.

In another preferred aspect Ar is indolonyl or substituted indolonyl.

In another preferred aspect Ar is benzimidazolyl or substituted benzimidazolyl.

In another preferred aspect Ar is quinolyl or substituted quinolyl.

In another preferred aspect Ar¹ is phenyl or substituted phenyl.

In another preferred aspect Ar¹ is pyridinyl or substituted pyridinyl.

In another preferred aspect Ar¹ is pyridazinyl or substituted pyridazinyl.

In another preferred aspect Ar¹ is pyrimidinyl or substituted pyrimidinyl.

In another preferred aspect Ar¹ is pyrazinyl or substituted pyrazinyl.

Preferred compounds of formula IV are:
(7R,9aS)-7-(4-fluorophenoxy)methyl-2-(5-chloro-pyridin-2-yl)-2,3,4,6,7,8,9,9a-octahydro-1*H-*pyrido[1,2-a]pyrazine;
(7R,9aS)-7-(3,5-difluorophenoxy)methyl-2-(5-chloro-pyridin-2-yl)-2,3,4,6,7,8,9,9a-octahydro-1 *H*-pyrido[1,2-a]pyrazine;
3-[(7R,9aS)-2-(5-chloro-pyridin-2-yl)-2,3,4,6,7,8,9,9a-octahydro-1*H*-pyrido[1,2-a]pyrazin-7-ylmethyl]-3*H*-benzooxazol-2-one;
3-[(7R,9aS)-2-(5-fluoro-pyrimidin-2-yl)-2,3,4,6,7,8,9,9a-octahydro-1*H*-pyrido[1,2-a]pyrazin-7-ylmethyl]-3*H*-benzoxazol-2-one;
(7R,9aS)-7-(4-fluorophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(3,5-difluorophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(3,4-dif)uorophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(3-cyanophenoxy)methyl-2-(5-fluoro-pyrimidin-2-yl)-2,3,4,6,7,8,9,9a-octahydro-1*H*-pyrido[1,2-a]pyrazine;
(7R,9aS)-7-(4-cyanophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(4-iodophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(4-fluorophenoxy)methyl-2-(4-fluorophenyl)-2,3,4,6,7,8,9,9a-octahydro-1*H*-pyrido[1,2-a]pyrazine;
(7S,9aS)-7-(4-fluorophenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(2-carbomethoxy-4-fluorophenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(2-bromo-4-fluorophenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(4-fluoro-2-trifluoromethylphenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3,5-difluorophenoxy)methyl-2-(5-chloro-pyridin-2-yl)-2,3,4,6,7,8,9,9a-octahydro-1 *H*-pyrido[1,2-a]pyrazine;
(7S,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluoro-2-methylphenoxy)methyl-2-(5-fluoro-pyrimidin-2-yl)-2,3,4,6,7,8,9,9a-octahydro- 1*H*-pyrido[1,2-a]pyrazine;
(7S,9aS)-7-(2,4-difluorophenoxy)methyl-2-(5-fluoro-pyrimidin-2-yl)-2,3,4,6,7,8,9,9a-octahydro-1*H*-pyrido[1,2-a]pyrazine;
(7S,9aS)-7-(3-methyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,4-difluoro-phenoxy)methyl-2-(5-fluoropyrimidin-2-yl)-2,3,4,6,7,8,9,9a-octahydro-1 H-pyrido[1,2-a]pyrazine;
(7S,9aS)-7-(3,5-difluoro-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-cyano-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-trifluoromethyl-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(4-trifluoromethyl-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-trifluoromethoxy-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-methoxy-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(4-methoxy-phenoxy)methyl-2-(5-fluoropyrimidin-2-yl)-2,3,4,6,7,8,9,9a-octahydro-1*H*-pyrido[1,2-a]pyrazine;
and pharmaceutically acceptable salts thereof.

This invention also relates to the pharmaceutically acceptable acid addition salts of compounds of the formula IV. The compounds of formula IV are basic in nature and are capable of forming a wide variety of salts with various inorganic and organic acids. The acids that may be used to prepare pharmaceutically acceptable acid addition salts of those compounds of formula IV are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, fumarate, gluconate, glucaronate, saccharate, formate, benzoate, glutamate, methanesulfonate, ethanesulfonate, benzenesulfonate, and p-toluenesulfonate.

The term "one or more substituents", as used herein, includes from one to the maximum number of substituents possible based on the number of available bonding sites.

The term "alkyl", as used herein, unless otherwise indicated, includes saturated monovalent hydrocarbon radicals having straight, branched or cyclic moieties or combinations thereof.

The term "alkoxy", as used herein, unless otherwise indicated, refers to radicals having the formula -O-alkyl, wherein "alkyl" is defined as above.

The compounds of formula IV above contain chiral centers and therefore exist in different enantiomeric forms. This invention relates to all optical isomers and all other stereoisomers of compounds of the formula IV and mixtures thereof.

Another example of D4 dopamine receptor antagonists that may be used in the methods and pharmaceutical compositions of this invention and compounds of the formula V 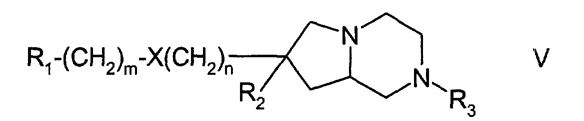 wherein
R₁ is phenyl, naphthyl, benzoxazolonyl, indolyl, indolonyl, benzimidazolyl, quinolyl, furyl, benzofuryl, thienyl, benzothienyl, oxazolyl, benzoxazolyl;
R₂ is H or (C₁-C₆)alkyl;
R₃ is phenyl, pyridinyl, pyrimidinyl, pyrazinyl, or pyridazinyl;
R₄ is H or (C₁-C₆)alkyl;
R₅ is H or (C₁-C₆)alkyl;
wherein each group of R₁ and R₃ may be independently and optionally substituted with one to four substituents independently selected from the groups consisting of fluoro, chloro, bromo, iodo, cyano, nitro, thiocyano, -SR₄, -SOR₄, -SO₂R₄, -NHSO₂R₄, -(C₁-C₆)alkoxy, -NR₄R₅, -NR₄COR₅, -CONR₄R₅, phenyl, -COR₄, -COOR₄, -(C₁-C₆)alkyl, -(C₁-C₆)alkyl substituted with one to six halogens, -(C₃-C₆)cycloalkyl, and trifluoromethoxy; X is O, S, SO, SO₂, NR₄, C=O, CH(OH), CHR₄, 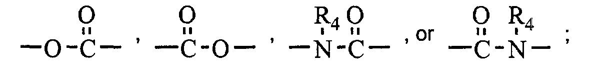
m is 0, 1 or 2;
n is 0, 1 or 2;
all stereoisomers thereof; or
a pharmaceutically acceptable salt thereof.

In addition the compounds of formula V wherein
R₁ is phenyl, naphthyl, benzoxazolonyl, indolyl, indolonyl, benzimidazolyl, or quinolyl;
wherein R₁ and R₃ may be independently substituted with up to three substituents independently selected from the group consisting of fluoro, chloro, bromo, iodo, cyano, -NR₄R₅, -(C₁-C₆)alkoxy, -COOR₄, -CONR₄R₅, -(C₁-C₆)alkyl, -(C₁-C₆)alkyl substituted with one to six halogens, -(C₃-C₆)cycloalkyl, and trifluoromethoxy;
R₂ is H or CH₃;
X is O, C=O, CHOH, -C(=O)O-, or CH₂;
m is 0 or 1;
n is 0 or 1; or
a pharmaceutically acceptable salt thereof.

Also the compounds of formula V wherein
R₁ is phenyl or substituted phenyl;
R₃ is substituted or unsubstituted phenyl, pyridinyl, or pyrimidinyl;
X is O, -C(=O)O-, or CH₂; or
a pharmaceutically acceptable salt thereof.

Also the compounds of formula V wherein
R₂ is H;
X is O;
m is 0;
n is 1; or
a pharmaceutically acceptable salt thereof.

Also the compounds of formula V wherein
R₂ is H;
X is O;
m is 1;
n is 0; or
a pharmaceutically acceptable salt thereof.

Also the compounds of formula V wherein
R₂ is H;
X is -C(=O)O-;
m is 0;
n is 0; or
a pharmaceutically acceptable salt thereof.

Also the compounds of formula V wherein
R₁ is fluorophenyl, diflurophenyl, or cyanophenyl;
R₃ is chloropyridinyl; or
a pharmaceutically acceptable salt thereof.

In addition the compounds of formula V wherein
R₁ is fluorophenyl, diflurophenyl, or cyanophenyl;
R₃ is fluoropyrimidinyl; or
a pharmaceutically acceptable salt thereof.

In addition the compounds of formula V wherein
R₁ is fluorophenyl, diflurophenyl, or cyanophenyl;
R₃ is chloropyridinyl; or
a pharmaceutically acceptable salt thereof.

In addition the compounds of formula V wherein
R₁ is fluorophenyl, diflurophenyl, or cyanophenyl;
R₃ is fluoropyrimidinyl; or
a pharmaceutically acceptable salt thereof.

In addition the compounds of formula V wherein
R₁ is fluorophenyl, diflurophenyl, or cyanophenyl;
R₃ is chloropyridinyl; or
a pharmaceutically acceptable salt thereof.

In addition the compounds of formula V wherein
R₁ is fluorophenyl, diflurophenyl, or cyanophenyl;
R₃ is fluoropyrimidinyl; or
a pharmaceutically acceptable salt thereof.

In addition the compounds of formula V wherein
R₃ is 5-chloro-pyridin-2-yl-; or
a pharmaceutically acceptable salt thereof.

In addition the compounds of formula V wherein
R₃ is 5-fluoro-pyrimidin-2-yl-; or
a pharmaceutically acceptable salt thereof.

In addition the compounds of formula V wherein
R₃ is 5-chloro-pyridin-2-yl-; or
a pharmaceutically acceptable salt thereof.

In addition the compounds of formula V wherein
R₃ is 5-fluoro-pyrimidin-2-yl-; or
a pharmaceutically acceptable salt thereof.

In addition the compounds of formula V wherein
R₃ is 5-chloro-pyridin-2-yl-; or
a pharmaceutically acceptable salt thereof.

In addition the compounds of formula V wherein
R₃ is 5-fluoro-pyrimidin-2-yl-; or
a pharmaceutically acceptable salt thereof.

Preferred compounds of the invention are:
(7S,8aS)-7-(4-fluorophenoxy)methyl-2-(5-chloropyridin-2-yl)-1,2,3,4,6,7,8,8a-octahydro-pyrrolo[1,2-a]pyrazine;
(7S,8aS)-7-(3,5-difluorophenoxy)methyl-2-(5-chloropyridin-2-yl)-1,2,3,4,6,7,8,8a-octahydro-pyrrolo[1 ,2-a]pyrazine;
(7S,8aS)-7-(3-cyanophenoxy)methyl-2-(5-chloropyidin-2-yl)-1,2,3,4,6,7,8,8a-octahydro-pyrrolo[1,2-a]pyrazine;
(7S,8aS)-7-(4-cyanophenoxy)methyl-2-(5-chloropyidin-2-yl)-1,2,3,4,6,7,8,8a-octahydro-pyrrolo[1 ,2-a]pyrazine;
(7S,8aS)-7-(4-fluorobenzyl)oxy-2-(5-chloropyridin-2-yl)-1,2,3,4,6,7,8,8a-octahydro-pyrrolo[1,2-a]pyrazine;
(7S,8aS)-2-(5-chloropyridin-2-yl)-1,2,3,4,6,7,8,8a-octahydro-pyrrolo[1,2-a]pyrazin-7-yl benzoate;
(7S,8aS)-7-(4-fluorophenoxy)methyl-2-(5-fluoropyrimidin-2-yl)-1,2,3,4,6,7,8,8a-octahydro-pyrrolo[1,2-a]pyrazine;
(7S,8aS)-7-(3,5-difluorophenoxy)methyl-2-(5-fluoropyrimidin-2-yl)-1,2,3,4,6,7,8,8a-octahydro-pyrrolo[1 ,2-a]pyrazine;
(7S,8aS)-7-(3-cyanophenoxy)methyl-2-(5-fluoropyrimidin-2-yl)-1,2,3,4,6,7,8,8a-octahydro-pyrrolo[1,2-a]pyrazine;
(7S,8aS)-7-(4-cyanophenoxy)methyl-2-(5-fluoropyrimidin-2-yl)-1,2,3,4,6,7,8,8a-octahydro-pyrrolo[1 ,2-a]pyrazine;
(7S,8aS)-7-(4-fluorobenzyl)oxy-2-(5-fluoropyrimidin-2-yl)-1,2,3,4,6,7,8,8a-octahydro-pyrrolo[1,2-a]pyrazine;
(7S,8aS)-2-(5-fluoropyrimidin-2-yl)-1,2,3,4,6,7,8,8a-octahydro-pyrrolo[1,2-a]pyrazin-7-yl benzoate;
(7S,8aS)-7-(3-cyanobenzyl)oxy-2-(5-fluoropyrimidin-2-yl)-1,2,3,4,6,7,8,8a-octahydro-pyrrolo[1,2-a]pyrazine;
and pharmaceutically acceptable salts thereof.

This invention also relates to the pharmaceutically acceptable acid addition salts of compounds of the formula V. The compounds of formula V are basic in nature and are capable of forming a wide variety of salts with various inorganic and organic acids. The acids that may be used to prepare pharmaceutically acceptable acid addition salts of those compounds of formula V are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, fumarate, gluconate, glucaronate, saccharate, formate, benzoate, glutamate, methanesulfonate, ethanesulfonate, benzenesulfonate, and p-toluenesulfonate.

The term "alkyl", as used herein, unless otherwise indicated, includes saturated monovalent hydrocarbon radicals having straight, branched or cyclic moieties or combinations thereof.

The term "alkoxy", as used herein, unless otherwise indicated, refers to radicals having the formula -O-alkyl, wherein "alkyl" is defined as above.

The compounds of formula V contain chiral centers and therefore exist in different enantiomeric forms. This invention relates to all stereoisomers of compounds of the formula **V** and mixtures thereof.

Another example of a D4 dopamine receptor antagonist that may be used in the methods and pharmaceutical compositions of this invention are compounds of the formula VI and VIA 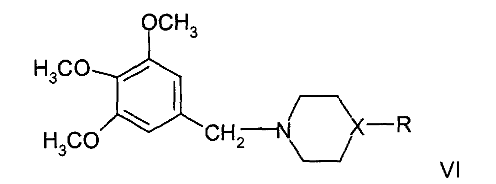
wherein X is N or CH; and
R is aryl or heteroaryl; or a pharmaceutically acceptable acid additional salt thereof; with the proviso that when x is N and R is aryl, aryl is not phenyl, phenyl monosubstituted by lower alkyl, lower alkoxy, halogen, or nitro, phenyl disubstituted by lower alkyl, or phenyl trisubstituted by lower alkoxy and formula VIA. 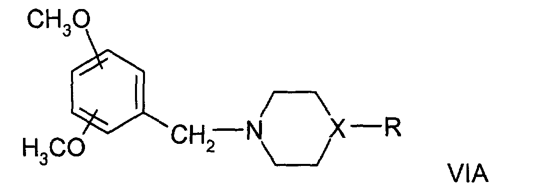
wherein X is N or CH; and
R is aryl or heteroaryl; or a pharmaceutically acceptable acid addition salt thereof; with the following provisos:
   (a) that when X is N or CH, and R is aryl, aryl is not phenyl, or phenyl monosubstituted by lower alkyl, lower alkoxy, or halogen; and
   (b) that when X is N and R is heteroaryl, heteroaryl is not 2-, 3-, or 4-pyridinyl.

In the compounds of formula VI or Formula VIA, the term "lower alkyl" means a straight or branched hydrocarbon radical having from 1 to 6 carbon atoms and includes, for example, methyl, ethyl, n-propyl, isopropyl, n-butyl, sec-butyl, isobutyl, tert-butyl, n-pentyl, n-hexyl, and the like.

The term "aryl" means an aromatic radical which a phenyl group or phenyl group substituted by 1 to 4 substituents selected from lower alkyl, lower alkoxy, lower thioalkoxy, halogen, nitro, amino, or cyano, such for as example, 2-chlorophenyl, 3-chlorophenyl, 4-chlorophenyl, 2-methylphenyl, 3-methylphenyl, 4-methylphenyl, 2-methoxyphenyl, 3-methoxyphenyl, 4-methoxyphenyl, 2-chloro-3-methylphenyl, 2-chloro-4-methylphenyl, 2-chloro-5-methylphenyl, 3-chloro-2-methylphenyl, 3-chloro-4-methylphenyl, 4-chloro 2-methylphenyl, 4-chloro-3-methylphenyl, 5-chloro-2-methylphenyl, 2,3-dichlorophenyl, 2,5-dichlorophenyl, 3,4-dichlorophenyl, 2,3-dimethylphenyl, 3,4-dimethylphenyl, and the like.

The term "heteroaryl" means a heteroaromatic radical which is 2-, 3- or 4-pyridinyl 4-,5-, 6-, or 7-benzo[b]furanyl, 4-, 5-,6-, or 7-benzo[b]thienyl, 4-,5-, 6-, or 7-indolyl, 2-, 3-, 4-,5-, 6-, 7-, or 8-quinolinyl, 2-, 3-,4-, 5-, 6-, 7-, or 8-isoquinolinyl.

"Lower alkoxy" and "lower thioalkoxy" are O-alkyl or S-alkyl of from 1 to 6 carbon atoms as defined above for "lower alkyl."

"Halogen", is fluorine, chlorine, bromine, or iodine.

A most preferred compound of Formula VI is one wherein

R is phenyl, phenyl substituted by 1 to 2 substituents selected from the group consisting of: lower alkyl, lower alkoxy, and halogen, or 2-pyridiny-I; with the proviso that when X is N,

R is not phenyl, phenyl monosubstituted by lower alkyl, lower alkoxy, or phenyl disubstituted by lower alkyl.

A most preferred compound of formula VI is one wherein

R is phenyl, phenyl substituted by 1 to 2 substituent selected from the group consisting of: methyl, methoxy, and chloro, or 2-pyridiny-I; with the proviso that when X is N,

R is not phenyl, phenyl monosubstituted by methyl, methoxy, and chloro, or phenyl disubstituted by methyl.

Preferred of Formula VI are:
1- (2, 5 -dichlorophenyl) - 4 - (3, 4, 5 - trimethoxyhenzyl) -piperazine;
1- (2, 3 -dichlorophenyl) - 4 - (3, 4, 5 - trimethoxybenzyl) -piperazine;
1 - (3, 4 -dichlorophenyl) - 4 - (3, 4, 5 - trimethoxybenzyl) -piperazine;
1-(2,3-dimethylphenyl)-4-(3,4,5-trimethoxybenzyl)-piperazine;
1-(3, 4 -dimethylphenyl) - 4 - (3, 4, 5 - trimethoxybenzyl) - piperazine;
1 - (2 - chloro - 3 -methyphenyl) - 4 - (3, 4, 5 - trimethoxybenzyl) piperazine;
1 - (2 - chloro - 4 -methyphenyl) - 4 - (3, 4, 5 - trimethoxybenzyl) piperazine;
1-(2-chloro-5-methylphenyl)-4-(3,4,5-trimethoxybenzyl) piperazine;
1-(3 - chloro- 2 -methyphenyl) - 4 - (3, 4, 5 - trimethoxvbenzyl) -piperazine;
1- (3 - chloro- 4 -methyphenyl) - 4 - (3, 4, 5 - trimethoxvbenzyl) -piperazine;
1- (5 - chloro - 2 -methyphenyl) - 4-; (3,4,5- trimethoxvbenzyl) -piperazine
1- (3-chloro-4-methylphenyl) -4- (3, 4, 5-trimethoxy-benzyl) piperazine;
1-(5-chloro-2-methylphenyl)-4-(3,4,5-trimethoxy-benzyl) piperazine;
1- (4 -chloro - 2 -methyphenyl) - 4 - (3, 4, 5 - trimethoxy -benzyl) piperazine;
1- (4 - chloro-3 -me thylphenyl) - 4 - (3, 4, 5 - trimethoxy -benzyl) piperazine;
1-pyridin-2-yl-4-(3,4,5-trimethoxybenzyl)-piperazine; and
4-phenyl-1-(3,4,5-trimethoxybenzyl)piperidine; or a pharmaceutically acceptable acid addition salt thereof.

Further preferred compounds of formula VI are:
1-phenyl-4- (3 4, 5-trrimethoxybenzyl) piperazine;
1-(2-chlorophenyl)-4-(3,4,5-trimethoxybenzyl)-piperazine;
1-(3-chlorophenyl)-4-(3,4,5-trimethoxybenzyl)-piperazine;
1-(4-chlorophenyl)-4-(3,4,5- trimethoxybenzyl)-piperazine;
1-o-tolyl-4- (3, 4, 5-trimethoxybenzyl) piperazine;
1-m-tolyl-4-(3,4,5-trimethoxybenzyl)piperazine;
1-m-tolyl-4-(3,4,5-trimethoxybenzyl)piperazine;
1-(2-methoxyphenyl)-4-(3,4,5-trimethoxybenzyl)-piperazine;
1-(3-methoxyphenyl)-4-(3,4,5-trimethoxybenzyl)-piperazine;
1-(4-methoxyphenyl)-4-(3,4,5-trimethoxybenzyl)-piperazine;
1-(2,5-dichlorophenyl)-4-(3,4,5-trimehoxybenzyl)-piperazine;
1 -(2,3-dichlorophenyl)-4-(1,4,5-trimethoxybenzyl)-piperazine;
1-(3,4-dichlorophenyl)-4-(3,4,5-trimethoxybenzyl)-piperazine;
1-(2,3-dimethylphenyl)-4-(3,4,5-trimethoxybenzyl)-piperazine;
1-(3,4-dimethylphenyl)-4-(3,4,5-trimethoxybenzyl)-piperazine;
1-(2-chloro-3-methylphenyl)-4--3,4,5-trimethoxybenzyl) piperazine;
1- (2-chloro-4-methylphenyl) -4-(3, 4, 5-trimethoxybenzyl) piperazine;
1-(2-chloro-5-methylphenyl)-4-(3,4,5-trimethoxy) benzyl) piperazine;
1- (3-chloro-2-methylphenyl) -4- (3, 4, 5-trimethoxybenzyl) piperazine;
1- (3-chloro-4-methylphenyl) -4- (3, 4, 5-trimethoxybenzyl) piperazine;
1-(5-chloro-2-methylphenyl)-4-(3,4,5-trime-hoxy-benzyl) piperazine;
1-(4-chloro-2-meLhylphenyl)-4-(3,4,5-trimethoxy-benzyl) piperazine;
1 -(4-chloro-3-methylohenyl )-4-(3,4,5-trimethoxy benzyl) piperazine;
1-pyridin-2-yl-4- (3,4,5-trimethoxybenzyl)-piperazine; and
4-phenyl-1- (3,4,5-trimethoxybenzyl) piperidine; or a pharmaceutically acceptable acid addition salt thereof.

A preferred compound of Formula VIA is one wherein
R is phenyl, phenyl substituted by 1 to 3 substituents selected from the group consisting of: lower alkyl, lower alkoxy, lower thioalkoxy, halogen, nitro, amino, and cyano,
2-, 3-, or 4-pyridinyl,
4-, 5-, 6-, or 7-benzo[b]furanyl,
4-,5-, 6-, or 7-benzo[b]thienyl,
4-,5-, 6-, or 7-indolyl,
2-, 3-, 4-, 5-, 6-, 7-, or 8-quinolinyl, or
2-, 3-, 4-, 5-, 6-, 7-, or 8-isoquinolinyl; with following provisos:
   (a) that when X is N or CH, R is not phenyl, or phenyl monosubstituted by lower alkyl, lower alkoxy, or halogen, and
   (b) that 'when X is N, R is not 2-, 3-, or 4-pyridinyl.

A more preferred compound of Formula VIA is one
wherein
R is phenyl, phenyl substituted by 1 to 2 substituents selected from the group consisting of: lower alkyl, lower alkoxy, and halogen, or 2-pyridinyl; with the -following provisos:
   (a) that when X is N or CH, R is not phenyl, phenyl monosubstituted by lower alkyl, lower alkoxy, or halogen, and
   (b) that when X is N, R is not 2-pyridinyl.

A most preferred compound of Formula VIA is one wherein R is-phenyl, phenyl substituted by 1 to 2 substituents selected from the group consisting of: methyl, methoxy, and chloro, or 2-pyridinyl; with the following provisos:
(a) that when X is N or CH, R is not phenyl, phenyl monosubstituted by methyl, methoxy ,and chloro ,and
(b) that when X is N, R. is not 2-,pyridinyl.

Particularly valuable compounds of Formula VIA are.
1- (2 -chloro- 3 -methyphenyl) - 4 - (2, 3 -dimethoxybenzyl) piperazine;
1-(2-chloro-3-methylphenyl)-4-(2,4-dimethoxybenzyl) piperazine;
1-(2-chloro-3-methylphenyl)-4-(2,5-dimethoxybenzyl) piperazine; and
1-(2-chloro-3 -methyphenyl)-4- (3, 4-dimethoxybenzyl) piperazine;
or a pharmaceutically acceptable acid addition salt thereof.

Furthermore, particularly valuable compounds of Formula Via used in the methods of the present invention are:
1- (2-chloro-3-methylphenyl) -4- (2, 3-dimethoxybenzyl) piperazine;
1- (2-chloro-3-methylphenyl) -4- (2, 4-dimethoxybenzyl) piperazine;
1(2-chloro-3-methylphenyl)-4-(2,5-dimethoxybenzyl) piperazine; and
1-(2-chloro-3-methylphenyl) -4-(3,4-dimethoxybenzyl), piperazine;
or a pharmaceutically acceptable acid addition salt thereof.

Another example of D4 dopamine receptor antagonists that may be used in the methods and pharmaceutical compositions of this invention are compounds of the formula VII, VIIA and VIIB. 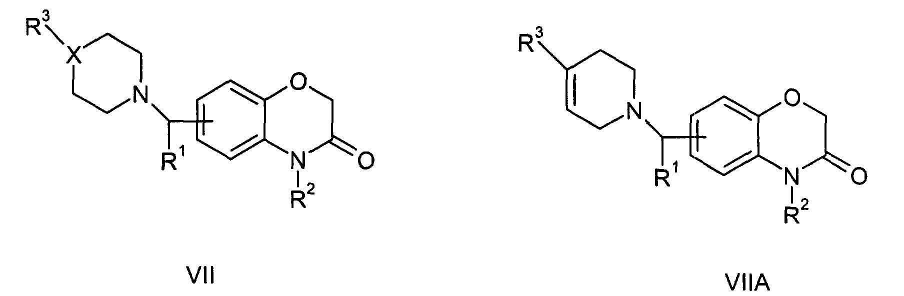
wherein R¹ and R² are independently hydrogen or C₁-C₆ alkyl;
X is N or CH; and
R³ is phenyl, naphthyl, heteroaryl, substituted phenyl, substituted naphthyl or substituted heteroaryl,
wherein each substituent is independently selected from halogen, C₁-C₆ alkoxy, C₁-C₆ alkyl, -CN, -CF₃, or sulphonamido, and the pharmaceutically acceptable salts, esters, amides, and prodrugs thereof.

Preferably in Formula VII or VIIA the group 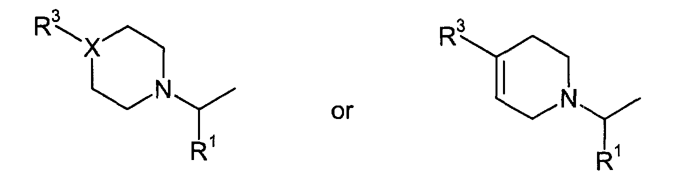 is attached to the benzoxazinone group at the 6 or 7 position.

Preferably R¹ and R² are hydrogen.

Preferably R³ is phenyl, methyltolyl, tolyl, or sulfonamido.

Preferably X is N.

Additionally there are compounds of formula VIIB, 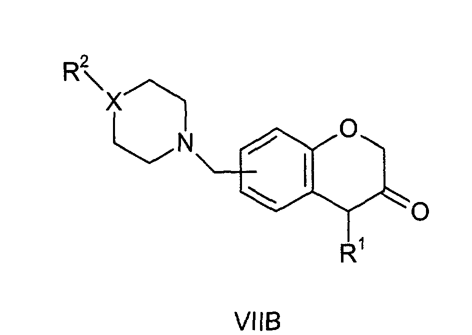 wherein X is N or CH; R¹ is hydrogen or methyl; and R² is phenyl or substituted phenyl wherein each substituent is independently selected from C₁-C₆ alkyl or sulphonamido, and the pharmaceutically acceptable salts, esters, amides, and a prodrugs thereof.

In a preferred embodiment of formula VIIB, the group, 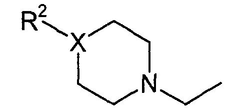 is attached to the benzoxazirione group at the 6 or 7 position.

In another preferred embodiment, R¹ is hydrogen.

In another preferred embodiment, R² is phenyl, methyltolyl, tolyl, or sulfonamido.

In a most preferred embodiment, the compounds of Formula VII, VIIA, and VIIB are:
4-(4-(3-oxo-3,4-dihydro-2H-benzo[1,4]oxazin-6-ylmethyl)-piperazin-1-yl]-benxenesulfonamide;
6-[4-(3, 4-dimethyl-phenyl)-piperazin-1-ylmethyl]-4H-benzo[1,4]oxazin-3-one;
6-(4-p-tolyl-piperazin-1-ylmethyl)-4H-benzo[1,4]oxazin-3-one;
6-[4-phenyl-piperazin-1-ylmethyl]-4H-benzo[1,4]oxazin-3-one;
7-(4-p-tolyl-piperazin-l-ylmethyl -4H-benzo[1,41oxazin-3-one;
7-(4-phenyl-piperazin-l-ylmethyl)-4H-benzo[1,4]oxazine-3-one;
7-[4-(3,4-dimethyl-phenyl)-piperazin-1-ylmethyl)-4H-benzo[1,4]oxazine-3-one;
6-[4-(5-methyl-pyrrdin-2-yl)-piperazin-1-ylmethyl]-4H-benzo[1,4]oxazin-3-one;
6-(4-p-tolyl-piperidin-1-ylmethyl)-4H-benxo1,4]oxazin-3-one;
6-[4-(3,4-Dimethyl-phenyl)piperidin-1-ylmethyl]-4H-benzo[1,4]oxazin-3-one;
6-(4-thiazol-2-yl-piperazin-1-ylmethyl)-4H-benzo(1,4]oxazin-3-one;
6-(4-benzothiazol-2-yl-piperazin-1-ylmethyl)-4H-benzo[1,4]oxazin.-3-one;
6-(4-(4,5-dimethyl-thiazol-2-yl)-piperazin-1-ylmethyl]-4H-benzo[1,4]oxazin-3-one;
6-(4-naphthalen-2-yl-piperazin-1-ylmethyl)-4H-benzo[1,4]oxazin-3-one;
6-[4-(3-chloro-phenyl)-piperazin-1-ylmethyl]-4H- benzo[1,4]oxazin-3-one;
6-[4-(3,4-dichloro-phenyl)-piperaziin-1-ylmethyl)-4H-benzo[1,4]oxazin-3-one;
2-[4-(3-oxo-3,4-dihydro-2H-benzo[1,4]oxazin-6-ylmethyl)-piperazin-1 -yl]-benzonitrile;
6-[4-(4-methoxy-phenyl)-piparazin-1-ylmethyl]-4H-benzo[1,4]oxazin-3-one;
6-[4-(2-chloro-4-methyl-phenyl)-piperazin-1-ylmethyl]-4H-benzo[1,4]oxazin-3-one;
6-(4-(4-Fluoro)-phenyl)-piperazin-1-ylmethyl]-4H-benzo[1,4]oxazin-3-one;
6-[4-(3-Trifluoromethyl-phenyl)-piperazin-1-ylmethyl]-4H-benzo[1,4]oxazin-3-one;
6-[4-(3,5-Dimethyl-phenyl)-piperaazin-1-ylmethyl]-4H-benzo[1,4]oxazin-3-one;
6-[4-(2-Chloro-phenyl)-piperazin-1-ylmethyl]-4H-benzo[1,4]oxazin-3-one;
6-[4-(4-Trifluoromethyl-phenyl)-piperazin-1-ylmethyl]-4H-benzo[1,4]oxazin-3-one;
6-[4-(4-Chloro-phenyl)-piperazin-1-ylmethyl]-4H-benzo[1,4]oxazin-3-one;
7-[4-(5-Methyl-pyridin-2-yl)-piperazin-1-ylmethyl]-4H-benzo[1,4]oxazin-3-one;
7-[4-(4-Methoxy-phenyl)-piperazin-1-yimethyl]-4H-benzo[1,4]oxazin-3-one,
7-[4-(4-Chloro-phenyl)-piperazin-1-ylmethyl]-4H-benzo[1,4]oxazin-3-one;
7-[4-(3,4-Dimethyl-phenyl)-piperidin-1-ylmethy]-4H-benzo[1,4]oxazin-3-one;
6-[4-(4-Methoxy-phenyl)-piperidin-1-ylmethyl]-4H-benzo(1,4]oxazin-3-one;
7-[4-(4-Methoxy-phenyl)-piperidin-1-ylmethyl]-4H-benzo(1,4]oxazin-3-one;
7-(4-Phenyl-piperidin-1-ylmethyl)-4H-benzo[1,4]oxazin-3-one;
7-(4-Naphthalen-2-yl-piperazin-1-ylmethy)-4H-benzo[1,4)oxazin-3-one; or
7-(4-p-Tolyl-piperidin-1-ylmethyl)-4H-benzo[1,4)oxazin-3-one.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The pharmaceutical compositions and methods of this invention comprise, or comprise administering D4 dopamine receptor antagonist of the formulas IV through VIIB, which may have chiral centers and therefore exist in different enantiomeric forms. This invention includes methods and pharmaceutical compositions, as described above, wherein the D4 dopamine receptor antagonists that are employed are optical isomers, tautomers or stereoisomers of the compounds of formulas IV through VIIB that are defined above, or mixtures thereof.

This present invention also relates to pharmaceutical compositions and methods comprising, or comprising administering, pharmaceutically acceptable acid addition salts of D4 dopamine receptor antagonists and of acetylcholine esterase inhibitors. The possible acids which are used to prepare the pharmaceutically acceptable acid addition salts of the basic active agents employed in the methods and pharmacuetical compositions of this invention are those which form non-toxic acid addition salts, *i.e.*, salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e*., 1,1'-methylene-bis-(2-hydroxy-3- naphthoate)]salts.

This invention also relates to pharmaceutical compositions and methods comprising, or comprising administering pharmaceutically acceptable base addition salts of D4 dopamine receptor antagonists and acetylcholine esterase inhibitors. The chemical bases that may be used as reagents to prepare pharmaceutically acceptable base salts of the acidic active agents that are employed in the methods of this invention are those that form non-toxic base salts with such compounds. Such non-toxic base salts include, but are not limited to those derived from such pharmacologically acceptable cations such as alkali metal cations (*e.g.*, potassium and sodium) and alkaline earth metal cations (*e.g.*, calcium and magnesium), ammonium or watersoluble amine addition salts such as N-methylglucamine (meglumine), and the lower alkanolammonium and other base salts of pharmaceutically acceptable organic amines.

The subject invention also relates to pharmaceutical compositions and methods of treatment that employ isotopically-labeled compounds that are identical to those recited in formulas IV through VIIB, or to other D4 dopamine receptor antagonists and formulas I through III or to other acetylcholine esterase inhibitors, but for the fact that one or more atoms are replaced by an atom having an atomic mass or mass number different from the atomic mass or mass number usually found in nature. Examples of isotopes that can be incorporated into the D4 dopamine receptor antagonists and the acetylcholine esterase inhibitors that are employed in the pharmaceutical compositions and methods of the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. The D4 dopamine receptor antagonists and the acetylcholine esterase inhibitors employed in the pharmaceutical compositions and methods of the present invention, prodrugs thereof, and pharmaceutically acceptable salts of said compounds or of said prodrugs which contain the aforementioned isotopes and/or other isotopes are within the scope of this invention. Certain isotopically-labeled D4 dopamine receptor antagonists and acetylcholine esterase inhibitors, for example, those into which radioactive isotopes such as ³H and ¹⁴C are incorporated, are useful in drug and/or substrate tissue distribution assays. Tritiated, *i.e.*, ³H, and carbon-14, *i.e.*, ¹⁴C, isotopes are particularly preferred for their ease of preparation and detectability. Further, substitution with heavier isotopes such as deuterium, *i.e.,* ²H, can afford certain therapeutic advantages resulting from greater metabolic stability, for example increased *in vivo* half-life or reduced dosage requirements and, hence, may be preferred in some circumstances.

### Detailed Description of the Invention

This invention relates both to methods of treating dementia or cognitive deficits associated with Alzheimer's Disease or Parkinson's Disease in which the D4 dopamine receptor antagonist and the acetylcholine esterase inhibitor, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the D4 dopamine receptor antagonist will be administered to an adult human in an amount ranging from about 0.01 to about 1000 mg per day, in single or divided doses, preferably from about 0.1 to about 100 mg/day. The compounds may be administered on a regimen of up to 6 times per day, preferably 1 to 4 times per day, especially 2 times per day and most especially once daily. A suitable dosage level for the acetylcholine esterase inhibitor is about 0.1 mg to 300 mg per day, preferably about 1 mg to 100 mg per day. Variations may nevertheless occur depending upon the species of mammal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The D4 dopamine receptor antagonists, their pharmaceutically acceptable salts, and the acetylcholine esterase inhibitors and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a D4 dopamine receptor antagonist and an acetylcholine esterase inhibitor, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc.* Moreover, oral pharmaceutical compositions can be suitably sweetened and/or flavored. In general, the therapeutic agents of this invention, when administered separately (*i*.*e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the D4 dopamine receptor antagonist and the acetylcholine esterase inhibitor may be formulated in a single pharmaceutical composition or alternatively in individual pharmaceutical compositions for simultaneous, separate or sequential use in accordance with the present invention.

Preferably the compositions according to the present invention, which contain both an D4 dopamine receptor antagonist and an acetylcholine inhibitor as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.*, conventional tabletting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from about 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavoured syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of a D4 dopamine receptor antagonist or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.,* Tween™ 20, 40, 60, 80 or 85) and other sorbitans (e.g., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn ™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a D4 dopamine receptor antagonist and an acetylcholine esterase inhibitor or pharmaceutically acceptable salts of the same, which process comprises bringing a D4 dopamine receptor antagonist and an acetylcholine esterase inhibitor or the pharmaceutically acceptable salts of one or both of these therapeutic agents into association with a pharmaceutically acceptable carrier or excipient.

It will be appreciated that the amount of the D4 dopamine receptor antagonist and the acetylcholineterase inhibitor required for use in the treatment of dementia or cognitive deficits associated with Alzheimer's disease and Parkinson's disease will vary not only with the particular compounds or compositions selected but also with the route of administration, the nature of the condition being treated, and the age and condition of the patient, and will ultimately be at the discretion of the patient's physician or pharmacist.

The utility of the compounds of the present invention as medical agents in the treatment of conditions which present with low cognitive function (e.g., Alzheimer's, Dementia) in mammals (e.g. humans) is demonstrated by the activity of the compounds of this invention in conventional assays and the in vitro assays described below in vitro estrogen receptor binding assay; and acetylcholinesterase inhibitor protocol. Such assays also provide a means whereby the activities of the compounds of this invention can be compared between themselves and with the activities of other known compounds. The results of these comparisons are useful for determining dosage levels in mammals, including humans, for the treatment of such diseases.

### Acetyl Cholinesterase inhibitor Protocol

Inhibition of Acetylcholinesterase (AChE) and Butyrylcholinesterase (BuChE). The method of Ellman, GL.; Courtney, K.D.; Andres, V., Jr.; Featherstone, R.M. A New and Rapid Colorimetric Determination of Acetylcholinesterase Activity. *Biochem. Pharmacol.* **1961,** 7, 88-95 was followed. The assay solution consists of a 0.1 M sodium phosphate buffer, pH 8.0, with the addition of 100 µM tetraisopropypyrophosphoramide (*iso*-OMPA), 100 µM 5,5'-dithiobis(2-nitrobenzoic acid) (DTNB), 0.02 units/mL AChE (Sigma Chemical Col, from human erythrocytes) and 200 µM acetylthiocholine iodide. The final assay volume was 0.25 mL. Test compounds were added to the assay solution prior to enzyme addition, whereupon a 20-min preincubation period with enzyme was followed by addition of substrate. Changes in absorbance at 412 nM were recorded for 5 min. The reaction rates were compared, and the percent inhibition due to the presence of test compounds was calculated.

Inhibition of butyrylcholinesterase was measured as described above for AChE by omitting addition of *iso*-OMPA and substitution 0.02 units/mL of BuChE (Sigma Chemical Co., from horse serum) and 200 µM butyrylthiocholine for enzyme and substrate, respectively.

*In vivo* Microdialysis. Male Sprague-Dawley rats were implanted in the corpus striatum with guide cannulae and dialysis probes (Bioanalytical Systems, West Lafayette, IN) and superfused at a rate 3 mL/minute. The dialysis fluid was a Ringer's buffer (pH 7.2) containing 500 nM physostigmine to reduce degradation of Ach by AChE. Fractions (60 µl) were collected every 20 minutes for 2 hours before drug administration and for 3 hours following oral administration of drug. Samples (50 µl) were used directly for HPLC analysis of Ach content as described above. Basal Ach release was defined as the average Ach content in the three fractions just prior to drug administration. Ach content in all fractions was converted to a percentage of these basal control values.

### D4 Receptor Binding Protocol

Dopaminergic activity is related to the ability of compounds to bind to mammalian dopamine receptors, and the relative ability of compounds of this invention to inhibit [³H]-spiperone binding to human dopamine D₄ receptor subtypes expressed in clonal cell lines was measured using the following procedure.

The determination of D₄ receptor binding ability has been described by Van Tol et al., *Nature* (London), **1991,** 350, 610. Clonal cell lines expressing the human dopamine D₄ receptor are harvested and homogenized (polytron) in a 50 mM Tris:HCl (pH 7.4 at 4°C) buffer containing 5 mM EDTA, 1.5 mM calcium chloride (CaCl₂), 5 mM magnesium chloride (MgCl₂), 5mM potassium chloride (KCI) and 120 mM sodium chloride (NaCI). The homogenates are centrifugated for 10-15 min. at 48,000 g, and the resulting pellets resuspended in a buffer at a concentration of 150-250 mg/ml. For saturation experiments, 0.75 ml aliquots of tissue homogenate are incubated in triplicate with increasing concentrations of [³H] spiperone (70.3 Ci/mmol; 10-3000 pM final concentration) for 30-120 minutes at 22°C in a total volume of 1 ml. For competition binding experiments, assays are initiated by the addition of 0.75 ml of membrane and incubated in duplicate with the indicated concentrations of competing ligands (10⁻¹⁴-10⁻³ M) and/or [³H]spiperone (100-300 pM) for 60-120 min at 22°C. Assays are terminated by rapid filtration through a Brandell cell harvester and the filters subsequently monitored for tritium as described by Sunahara, R.K. et. al., *Nature* (London), **1990,** 346, 76. For all experiments, specific [³H]spiperone binding is defined as that inhibited by 1-10 mM (+)-butaclamol. Binding data are analyzed by non-linear least square curve-fitting.

When administered in combination, either as a single or as separate pharmaceutical composition(s), the D4 dopamine receptor antagonist and the acetylcholine esterase inhibitor, are presented in a ratio which is consistent with the manifestation of the desired effect. In particular, the ratio by weight of the D4 dopamine receptor antagonist and an acetylcholine esterase inhibitor will suitably be between 0.001 and 1 to 1000 to 1, and especially between 0.01 to 1 and 100 to 1.

## Claims

1. A pharmaceutical composition for the treatment of dementia or cognitive deficits associated with Alzheimer's Disease or Parkinson's Disease in a mammal, comprising: (a) a D4 dopamine receptor antagonist or a pharmaceutically acceptable salt thereof; (b) an acetylcholine esterase inhibitor or pharmaceutically acceptable salt thereof; and (c) a pharmaceutically acceptable carrier; wherein the active agents "a" and "b" above are present in amounts that render the composition effective in treating, respectively, dementia or cognitive deficits associated with Alzheimer's disease or Parkinson's disease.

2. A pharmaceutical composition according to claim 1, wherein the acetylcholine esterase inhibitor or pharmaceutically acceptable salt thereof is selected from compounds of the formula I, as defined below, and their pharmaceutically acceptable salts: 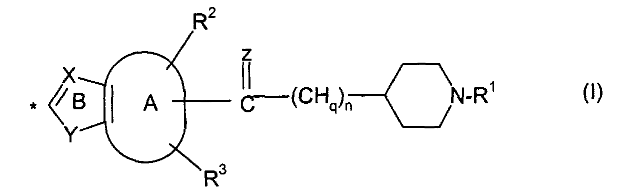 wherein one of R², R³ and the side chain containing 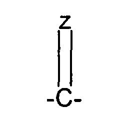
may optionally be attached to the carbon atom designated by an asterisk in ring B rather than to a member of ring A;
ring A is benzo, thieno, pyrido, pyrazino, pyrimido, furano, seleno, pyrrolo, thiazolo, or imidazolo;
R¹ is phenyl, phenyl-(C₁-C₆)alkyl, cinnamyl or heteroarylmethyl, wherein the heteroaryl moiety of said heteroarylmethyl is selected from imidazolo, thiazolo, thieno, pyrido and isoxazolo, and wherein said phenyl and said heteroaryl moiety may optionally be substituted with one or two substituents independently selected from (C₁-C₆)alkyl, (C₁-C₆)alkoxy and halo;
R² and R³ are independently selected from hydrogen, (C₁-C₆)alkoxy, (C₁-C₆)alkyl optionally substituted with from one to three fluorine atoms, benzyloxy, hydroxy, phenyl, benzyl, halo, nitro, cyano, COOR⁴, CONHR⁴, NR⁴R⁵, NR⁴COR⁵, or SOₚCH₂-phenyl wherein p is 0, 1 or 2;
or R² and R³ are attached to adjacent carbon atoms and form, together with the carbons to which they are attached, a five or six membered ring wherein each atom of the ring is carbon, nitrogen or oxygen (e.g., a methylenedioxy, ethylenedioxy or lactam ring);
R⁴ and R⁵ are independently selected from hydrogen and (C₁-C₆)alkyl, or R⁴ and R⁵, when part of said NR⁴R⁵, optionally form, together with the nitrogen to which they are attached, a ring containing four to eight members wherein one atom of the ring is nitrogen and the others are carbon, oxygen or nitrogen, or R⁴ and R⁵, when part of said NR⁴COR⁵, optionally form, together with the nitrogen and carbon to which they are attached, a four to eight membered lactam ring;
X is nitrogen or CH;
Y is oxygen, sulfur or NR⁶;
R⁶ is hydrogen, (C₁-C₆)alkyl, CO(C₁-C₆)alkyl or SO₂-phenyl, wherein the phenyl moiety of said SO₂-phenyl may optionally be substituted with from one to five substituents independently selected from (C₁-C₄) alkyl;
n is an integer from 1 to 4;
each q is independently 1 or 2; and
Z is oxygen or sulfur;
with the proviso that any CH_{q} group wherein q is 1 must be attached to one and only one other CH_{q} group wherein q is 1.

3. A pharmaceutical composition according to claim 1, wherein the acetylcholine esterase inhibitor or pharmaceutically acceptable salt thereof is selected from compounds of the formula II, as defined below, and their pharmaceutically acceptable salts: 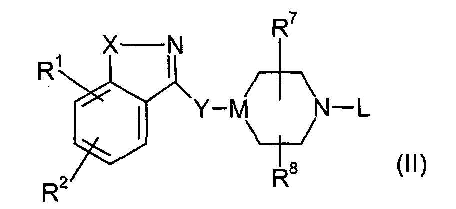
wherein R¹ and R² are independently selected from hydrogen, (C₁-C₆)alkoxy, benzyloxy, phenoxy, hydroxy, phenyl, benzyl, halo, nitro, cyano, -COOR⁵, -CONHR⁵, -NR⁵R⁶, -NR⁵COR⁶, -OCONR⁵R⁶, -NHCOOR⁵, (C₁-C₆)alkyl optionally substituted with from 1 to 3 fluorine atoms; SOₚCH₂-phenyl or SOₚ(C₁-C₆)alkyl, wherein p is 0, 1 or 2; pyridylmethyloxy or thienylmethyloxy; wherein the phenyl moieties of said phenoxy, benzyloxy, phenyl and benzyl groups, and the pyridyl and thienyl moieties of said pyridylmethyloxy and thienylmethyloxy may optionally be substituted with 1 or 2 substituents independently selected from halo, (C₁-C₄)alkyl, trifluoromethyl, (C₁-C₄)alkoxy, cyano, nitro and hydroxy; 2-oxazolyl, 2-thiazolyl and benzenesulfonamide;
or R¹ and R², when attached to adjacent carbon atoms and when X is oxygen or sulfur may form, together with the carbon atoms to which they are attached, a group of the formula 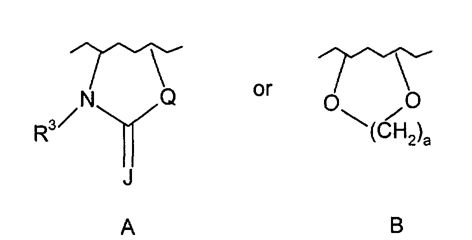
wherein J is oxygen, sulfur or NR⁴ wherein R⁴ is hydrogen or (C₁-C₄)alkyl, "a" is 1 or 2, R³ is hydrogen or (C₁-C₄)alkyl and Q is oxygen, sulfur, NH, CHCH₃, C(CH₃)₂, -CH=CH-, or (CH₂)₁ wherein I is an integer from 1 to 3;
X is oxygen, sulfur, -CH=CH-, -CH=N-, -N=CH-, -N=N-, or NR⁴ wherein R⁴ is hydrogen or (C₁-C₄) alkyl;
Y is -(CH₂)ₘ-, -CH=CH(CH₂)ₙ-, -NR⁴(CH₂)ₘ-, Or -O(CH₂)ₘ-
wherein R⁴ is defined as above, n is an integer from 0 to 3 and m is an integer from 1 to 3;
R⁵ and R⁶ are each independently selected from hydrogen, (C₁-C₆)alkyl, phenyl or benzyl, wherein the phenyl moieties of said phenyl and benzyl may optionally be substituted with 1 or 2 substituents independently selected from fluoro, chloro, bromo, iodo, (C₁-C₄) alkyl, trifluoromethyl, (C₁-C₄) alkoxy, cyano, nitro and hydroxy, or NR⁵R⁶ together form a 4 to 8 membered ring wherein one atom of the ring is nitrogen and the others are carbon, oxygen or nitrogen (e.g. pyrrolidinyl, piperidinyl, morpholino, piperazinyl or N-methylpiperazinyl), or NR⁵COR⁶ together form a 4 to 8 membered cyclic lactam ring;
M is -CH- or nitrogen;
L is phenyl, phenyl-(C₁-C₆)alkyl, cinnamyl or pyridylmethyl, wherein the phenyl moieties of said phenyl and phenyl-(C₁-C₆)alkyl may optionally be substituted with 1 to 3 substituents independently selected from (C₁-C₆)alkyl, (C₁-C₆)alkoxy, (C₁-C₄)alkoxycarbonyl, (C₁-C₄)alkylcarbonyl or halo; or L is a group of the formula 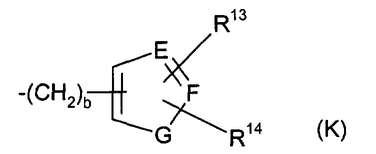
wherein b is an integer from 1 to 4, R¹³ and R¹⁴ are independently selected from hydrogen, (C₁-C₄) alkyl, halo and phenyl, E and F are independently selected from -CH- and nitrogen, and G is oxygen, sulfur or NR⁴ wherein R⁴ is defined as above, with the proviso that when E and F are both nitrogen, one of R¹³ and R¹⁴ is absent; and
R⁷ and R⁸ are independently selected from hydrogen, (C₁-C₆)alkyl, (C₁-C₆)alkoxycarbonyl, (C₁-C₆)alkylcarbonyl and (C₁-C₆)alkoxy, with the proviso that said (C₁-C₆)alkoxy is not attached to a carbon that is adjacent to a nitrogen.

4. A pharmaceutical composition according to claim 1, wherein acetylcholine esterase inhibitor or pharmaceutically acceptable salt thereof is selected from compounds of the formula III: 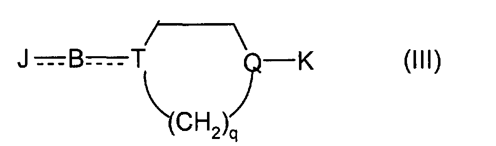
in which J is
(a) a group, substituted or unsubstituted, selected from the group consisting of (1) phenyl, (2) pyridyl, (3) pyrazyl, (4) quinolyl, (5) cyclohexyl, (6) quinoxalyl and (7) furyl;
(b) a monovalent or divalent group, in which the phenyl may have a substituent(s), selected from the group consisting of (1) indanyl, (2) indanonyl, (3) indenyl, (4) indenonyl, (5) indanedionyl, (6) tetralonyl, (7) benzosuberonyl, (8) indanolyl and (9) C₆ H₅ -CO-CH(CH₃)-;
(c) a monovalent group derived from a cyclic amide compound;
(d) a lower alkyl or
(e) a group of R²¹ -CH=CH- in which R²¹ is hydrogen or a lower alkoxycarbonyl;
B is -(CHR²²)ᵣ-, -CO-(CHR²²)ᵣ-, -NR⁴-(CHR²²)ᵣ-, R⁴ being hydrogen, a lower alkyl, an acyl, a lower alkylsulfonyl, phenyl, a substituted phenyl, benzyl or a substituted benzyl, -CO-NR⁵-(CHR²²)ᵣ -, R⁵ being hydrogen, a lower alkyl or phenyl, -CH=CH-(CHR²²)ᵣ -, -OCOO-(CHR²²)ᵣ-, -OOC-NH-(CHR²²)ᵣ-, -NH-CO-(CHR²²)ᵣ -, -CH₂-CO-NH-(CHR²²)ᵣ-, -(CH₂)₂ -NH-(CHR²²)ᵣ -, -CH(OH)-(CHR²²)ᵣ-, r being zero or an integer of 1 to 10, R²² being hydrogen or methyl so that one alkylene group may have no methyl branch or one or more methyl branch, =(CH-CH=CH)_{b}-, b being an integer of 1 to 3, =CH-(CH₂)_{c} -, c being zero or an integer of 1 to 9, (CH-CH)_{d}=, d being zero or an integer of 1 to 5; -CO―CH=CH-CH₂-, -CO-CH₂-CH(OH)-CH₂-, -CH(CH₃)-CO-NH-CH₂-, -CH=CH-CO-NH-(CH₂)₂-, -NH-, -O-, -S-, a dialkylaminoalkylcarbonyl or a lower alkoxycarbonyl;
T is a nitrogen or carbon;
Q is nitrogen, carbon or 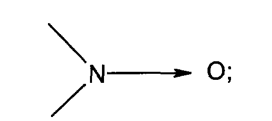
q is an integer of 1 to 3;
K is hydrogen, phenyl, a substituted phenyl, an arylalkyl in which the phenyl may have a substituent, cinnamyl, a lower alkyl, pyridylmethyl, a cycloalkylalkyl, adamantanemethyl, furylmenthyl, a cycloalkyl, a lower alkoxycarbonyl or an acyl; and shows a single bond or a double bond.

5. A pharmaceutical composition according to claim 1, wherein the D4 dopamine receptor antagonist or pharmaceutically acceptable salt thereof is selected from compounds of the formula IV: 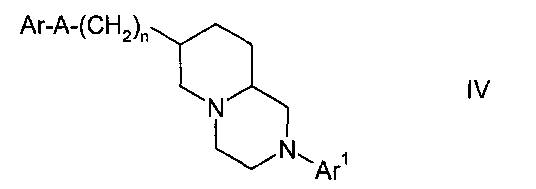
wherein Ar is phenyl, naphthyl, benzoxazolonyl, indolyl, indolonyl, benzimidazolyl, quinolyl, furyl, benzofuryl, thienyl, benzothienyl, oxazolyl, or benzoxazolyl;
Ar¹ is phenyl, pyridinyl, pyridazinyl, pyrimidinyl, or pyrazinyl;
A is O, S, SO, SO₂, C=O, CHOH, or -(CR³R⁴)-;
n is 0, 1 or 2;
each of Ar and Ar¹ may be independently and optionally substituted with one to four substituents independently selected from the group consisting of fluoro, chloro, bromo, iodo, cyano, nitro, thiocyano, -SR, -SOR, -SO₂R, -NHSO₂R, -(C₁-C₆)alkoxy, -NR¹R², -NRCOR¹, -CONR¹R², Ph, -COR, COOR, -(C₁-C₆)alkyl, -(C₁-C₆)alkyl substituted with one to six halogens, -(C₃-C₆)cycloalkyl, and trifluoromethoxy;
each and every R, R¹, and R² is independently selected from the group consisting of hydrogen, -(C₁-C₆)alkyl, -(C₁-C₆)alkyl substituted with one to thirteen halogens selected from fluorine, chlorine, bromine and iodine, phenyl, benzyl, -(C₂-C₆)alkenyl, -(C₃-C₆)cycloalkyl, and -(C₁-C₆)alkoxy;
each and every R³ and R⁴ is independently selected from the group consisting of hydrogen, methyl, ethyl, n-propyl, or i-propyl;
diastereomeric and optical isomers thereof; and
pharmaceutically acceptable salts thereof.

6. A pharmaceutical composition according to claim 1, wherein the D4 dopamine receptor antagonist or pharmaceutically acceptable salt thereof is selected from compounds of the formula V: 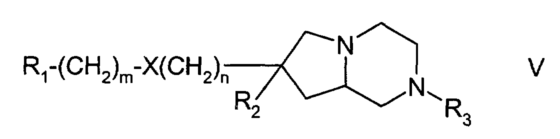 wherein
R₁ is phenyl, naphthyl, benzoxazolonyl, indolyl, indolonyl, benzimidazolyl, quinolyl, furyl, benzofuryl, thienyl, benzothienyl, oxazolyl, benzoxazolyl;
R₂ is H or (C₁-C₆)alkyl;
R₃ is phenyl, pyridinyl, pyrimidinyl, pyrazinyl, or pyridazinyl;
R₄ is H or (C₁-C₆)alkyl;
R₅ is H or (C₁-C₆)alkyl;
wherein each group of R₁ and R₃ may be independently and optionally substituted with one to four substituents independently selected from the groups consisting of fluoro, chloro, bromo, iodo, cyano, nitro, thiocyano, -SR₄, -SOR₄, -SO₂R₄, -NHSO₂R₄, -(C₁-C₆)alkoxy, -NR₄R₅, -NR₄COR₅, -CONR₄R₅, phenyl, -COR₄, -COOR₄, -(C₁-C₆)alkyl, -(C₁-C₆)alkyl substituted with one to six halogens, -(C₃-C₆)cycloalkyl, and trifluoromethoxy; X is O, S, SO, SO₂, NR₄, C=O, CH(OH), CHR₄, 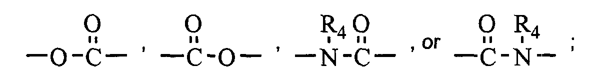
m is 0, 1 or 2;
n is 0, 1 or 2;
all stereoisomers thereof; or
a pharmaceutically acceptable salt thereof.

7. A pharmaceutical composition according to claim 1, wherein the D4 dopamine receptor antagonist pharmaceutically acceptable salt thereof is selected from compounds of the formula VI and VIA: 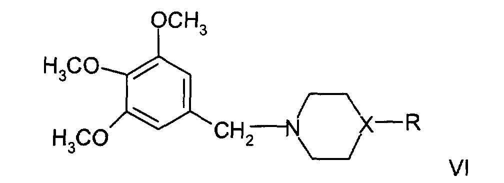
wherein X is N or CH; and
R is aryl or heteroaryl; or a pharmaceutically acceptable acid additional salt thereof; with the proviso that when X is N and R is aryl, aryl is not phenyl, phenyl monosubstituted by lower alkyl, lower alkoxy, halogen, or nitro, phenyl disubstituted by lower alkyl, or phenyl trisubstituted by lower alkoxy and formula VIA. 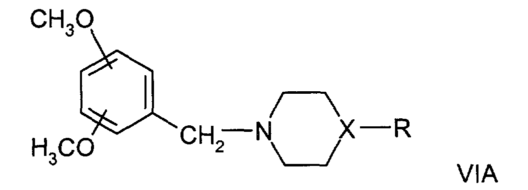
wherein X is N or CH; and
R is aryl or heteroaryl; or a pharmaceutically acceptable acid addition salt thereof; with the following provisos:
(a) that when X is N or CH, and R is aryl, aryl is not phenyl, or phenyl monosubstituted by lower alkyl, lower alkoxy, or halogen; and
(b) that when X is N and R is heteroaryl, heteroaryl is not 2-, 3-, or 4-pyridinyl.

8. A pharmaceutical composition according to claim 1, wherein the D4 dopamine receptor antagonist or pharmaceutically acceptable salt thereof is selected from compounds of the formula VII and VIIA: 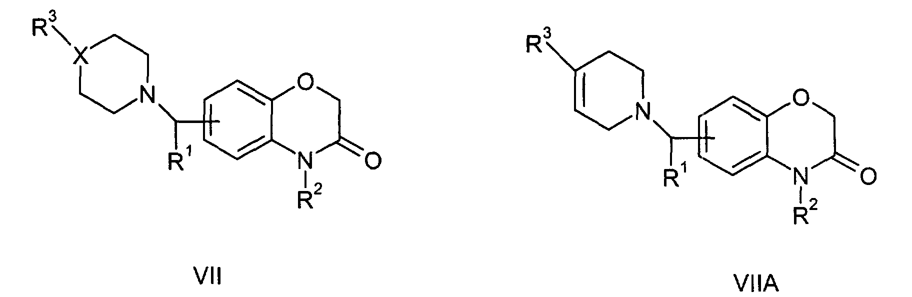
wherein R¹ and R² are independently hydrogen or C₁-C₆ alkyl;
X is N or CH; and
R³ is phenyl, naphthyl, heteroaryl, substituted phenyl, substituted naphthyl or substituted heteroaryl,
wherein each substituent is independently selected from halogen, C₁-C₆ alkoxy, C₁-C₆ alkyl, -CN, -CF₃, or sulphonamido, and the pharmaceutically acceptable salts, esters, amides, and prodrugs thereof and wherein in formula VII or VIIA the group 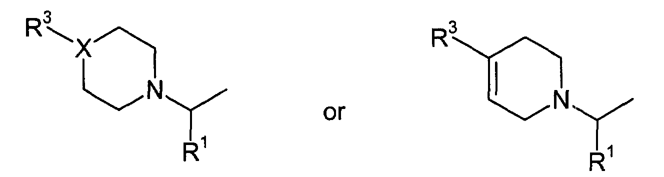 is attached to the benzoxazinone group at the 6 or 7 position.

9. A pharmaceutical composition according to claim 1, wherein the D4 dopamine receptor antagonist or pharmaceutically acceptable salt thereof is selected from compounds of the formula VIIB: 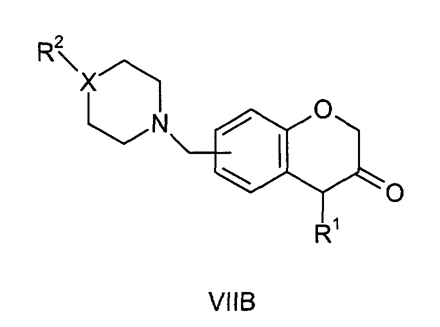 wherein X is N or CH; R¹ is hydrogen or methyl; and R² is phenyl or substituted phenyl wherein each substituent is independently selected from C₁-C₆ alkyl or sulphonamido, and the pharmaceutically acceptable salts, esters, amides, and a prodrugs thereof and the group 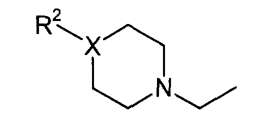 is attached to benzoxazirione group at the 6 or 7 position.

10. A method of treating dementia or cognitive deficits associated with a Alzheimer's Disease or Parkinson's Disease in a mammal, comprising administering to said mammal: (a) a D4 dopamine receptor antagonist or a pharmaceutically acceptable salt thereof; and (b) an acetylcholine esterase inhibitor, or pharmaceutically acceptable salt thereof; wherein the active agents "a" and "b" above are present in amounts that render the combination of the two agents effective in treating, respectively, dementia or cognitive deficits associated with Alzheimer's Disease or Parkinson's Disease.

11. A method according to claim 10, wherein the acetylcholine esterase inhibitor or pharmaceutically acceptable salt thereof that is employed in such a method is selected from the following compounds:
1-(2-methyl-1H-benzimidazol-5-yl)-3-[1-(phenylmethyl)-4-piperidinyl]-1-propanone;
1-(2-phenyl-1H-benzimidazol-5-yl)-3-[1-(phenylmethyl)-4-piperidinyl]-1-propanone;
1-(1-ethyl-2-methyl-1H-benzimidazol-5-yl)-3-[1-(phenylmethyl)-4-piperidinyl]-1-propanone;
1-(2-methyl-6-benzothiazolyl)-3-[1-(phenylmethyl)-4-piperidinyl]-1-propanone;
1-(2-methyl-6-benzothiazolyl)-3-[1-[(2-methyl-4-thiazolyl)methyl]-4-piperidinyl]-1-propanone;
1-(5-methyl-benzo[b]thien-2-yl)-3-[1-(phenylmethyl)-4-piperidinyl]-1-propanone;
1-(6-methyl-benzo[b]thien-2-yl)-3-[1-(phenylmethyl)-4-piperidinyl]-1-propanone;
1-(3,5-dimethyl-benzo[b]thien-2-yl)-3-[1-(phenylmethyl)-4-piperidinyl]-1-propanone;
1 -(benzo[b]thien-2-yl)-3-[1-(phenylmethyl)-4-piperidinyl]-1-propanone;
1-(benzofuran-2-yl)-3-[1-(phenylmethyl)-4-piperidinyl]-1-propanone;
1-(1-phenylsulfonyl-6-methyl-indol-2-yl)-3-[1-(phenylmethyl)-4-piperidinyl]-1-propanone;
1-(6-methyl-indol-2-yl)-3-[1-(phenylmethyl)-4-piperidinyl]-1-propanone;
1 -(1 -phenylsulfonyl-5-amino-indol-2-yl)-3-[1 -(phenylmethyl)-4-piperidinyl]-1 -propanone;
1-(5-amino-indol-2-yl)-3-[1-(phenylmethyl)-4-piperidinyl]-1-propanone; and
1-(5-acetylamino-indol-2-yl)-3-[1-(phenylmethyl)-4-piperidinyl]-1-propanone.
1-(6-quinolyl)-3-[1-(phenylmethyl)-4-piperidinyl]-1-propanone;
1-(5-indolyl)-3-[1-(phenylmethyl)-4-piperidinyl]-1-propanone;
1-(5-benzthienyl)-3-[1-(phenylmethyl)-4-piperidinyl]-1-propanone;
1-(6-quinazolyl)-3-[1-(phenylmethyl)-4-piperidinyl]-1-propanone;
1-(6-benzoxazolyl)-3-[1-(phenylmethyl)-4-piperidinyl]-1-propanone;
1-(5-benzofuranyl)-3-[1-(phenylmethyl)-4-piperidinyl]-1-propanone;
1-(5-methyl-benzimidazol-2-yl)-3-[1-(phenylmethyl)-4-piperidinyl]-1-propanone;
1-(6-methyl-benzimidazol-2-yl)-3-[1-(phenylmethyl)-4-piperidinyl]-1-propanone;
1-(5-chloro-benzo[b]thien-2-yl)-3-[1-(phenylmethyl)-4-piperidinyl]-1-propanone;
1-(5-azaindol-2-yl)-3-[1-(phenylmethyl)-4-piperidinyl]-1-propanone;
1-(6-azabenzo[b]thien-2-yl)-3-[1-(phenylmethyl)-4-piperidinyl]-1-propanone;
1-(1H-2-oxo-pyrrolo[2N,3N,5,6]benzo[b]thieno-2-yl)-3-[1-(phenylmethyl)-4-piperidinyl]-1-propanone;
1-(6-methyl-benzothiazol-2-yl)-3-[1-(phenylmethyl)-4-piperidinyl]-1-propanone;
1-(6-methoxy-indol-2-yl)-3-[1-(phenylmethyl)-4-piperidinyl]-1-propanone;
1-(6-methoxy-benzo[b]thien-2-yl)-3-[1-(phenylmethyl)4-piperidinyl]-1-propanone;
1-(6-acetylamino-benzo[b]thien-2-yl)-3-[1-(phenylmethyl)-4-piperidinyl]-1 -propanone; and 1-(5-acetylamino-benzo[b]thien-2-yl)-3-[1-(phenylmethyl)4-piperidinyl]-1-propanone.

12. A method according to claim 10, wherein the acetylcholine esterase inhibitor that is employed in such a method is selected from the following compounds and their pharmaceutically acceptable salts:
6-hydroxy-3-[2-[1-(phenylmethyl)-4-piperidinyl]ethyl]-1,2-benzisoxazole;
5-methyl-3-[2-[1-(phenylmethyl)-4-piperidinyl]ethyl]-1,2-benzisoxazole;
6-methoxy-3[2-[-1(phenylmethyl)-4-piperidinyl]ethyl]-1,2-benzisoxazole;
6-acetamido-3-[2-[1-(phenylmethyl)-4-piperidinyl]-ethyl]-1,2-benzisoxazole;
6-amino-3-[2-[1-(phenylmethyl)-4-piperidinyl]ethyl]-1, 2-benzisoxazole;
6-(4-morpholinyl)-3-[2-[1-(phenylmethyl)-4-piperidinyl]ethyl]-1,2-benzisoxazole;
5,7-dihydro-3-[2-[1-(phenylmethyl)-4-piperidinyl]ethyl]-6H-pyrrolo[4,5-f]-1,2-benzisoxazol-6-one;
3-[2-[1-(phenylmethyl)-4-piperidinyl]ethyl]-1,2-benzisothiazole;
3-[2-[1-(phenylmethyl)-4-piperidinyl]ethenyl]-1,2-benzisoxazole;
6-phenylamino-3-[2-[1-(phenylmethyl)-4-piperidinyl]ethyl]-1,2,-benzisoxazole;
6-(2-thiazoly)-3-[2-[1-(phenylmethyl)-4-piperidinyl]ethyl]-1,2-benzisoxazole;
6-(2-oxazolyl)-3-[2-[1-(phenylmethyl)-4-piperidinyl]ethyl]-1,2-benzisoxazole;
6-pyrrolidinyl-3-[2-[1-(phenylmethyl)-4-piperidinyl]ethyl]-1,2-benzisoxazole;
5,7-dihydro-5,5-dimethyl-3-[2-[1-(phenylmethyl)-4-piperidinyl]ethyl]-6H-pyrrolo[4,5-f]-1,2-benzisoxazole-6-one;
6,8-dihydro-3-[2-[1-(phenylmethyl)-4-piperidinyl]ethyl]-7H-pyrrolo[5,4-g]-1,2-benzisoxazole-7-one; and
3-[2-[1-(phenylmethyl)-4-piperidinyl]ethyl]-5,6,8-trihydro-7H-lsoxazolo[4,5-g]-quinolin-7-one.

13. A method according to claim 10, wherein the acetylcholine esterase inhibitor or pharmaceutically acceptable salt thereof that is employed in such a method is selected from the following compounds and their pharmaceutically acceptable salts:
1-benzyl-4-((5,6-dimethoxy-1-indanon)-2-yl)methylpiperidine,
1-benzyl-4-((5,6-dimethoxy-1-indanon)-2-ylidenyl)methylpiperidine,
1-benzyl-4-((5-methoxy-1 -indanon)-2-yl)methylpiperidine,
1-benzyl-4-((5,6-diethoxy-1-indanon)-2-yl)methylpiperidine,
1-benzyl-4-((5,6-methnylenedioxy-1-indanon)-2-yl)methylpiperidine,
1-(m-nitrobenzyl)-4-((5,6-dimethoxy-1-indanon)-2-yl)methylpiperidine,
1-cyclohexymethyl-4-((5,6-dimethoxy-1-indanon)-2-yl)methylpiperidine,
1-(m-florobenzyl)-4-((5,6-dimethoxy-1-indanon)-2-yl)methylpiperidine,
1-benzyl-4-((5,6-dimethoxy-1-indanon)-2-yl)propylpiperidine,
1-benzyl-4-((5-isopropoxy-6-methoxy-1-indanon)-2-yl)methylpiperidine and
1-benzyl-4-((5,6-dimethoxy-1-indanolidenyl)-2-yl)propenylpiperidine, having the below shown formula,

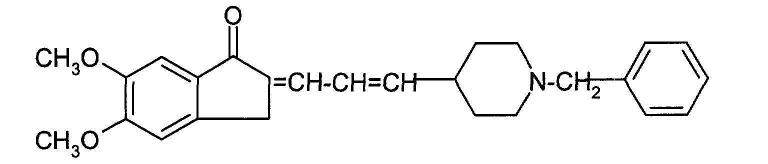

14. A method according to claim 10, wherein the D4 dopamine receptor antagonist that is employed in such a method is selected from the following compounds and their pharmaceutically acceptable salts:
(7R,9aS)-7-(4-fluorophenoxy)methyl-2-(5-chloro-pyridin-2-yl )-2,3,4,6,7,8,9,9a-octahydro-1 *H*-pyrido[1,2-a]pyrazine;
(7R,9aS)-7-(3,5-difluorophenoxy)methyl-2-(5-chloro-pyridin-2-yl)-2,3,4,6,7,8,9,9a-octahydro-1 *H*-pyrido[1,2-a]pyrazine;
3-[(7R,9aS)-2-(5-chloro-pyridin-2-yl)-2,3,4,6,7,8,9,9a-octahydro-1*H*-pyrido[1 ,2-a]pyrazin-7-ylmethyl]-3*H*-benzooxazol-2-one;
3-[(7R,9aS)-2-(5-fluoro-pyrimidin-2-yl)-2,3,4,6,7,8,9,9a-octahydro-1*H*-pyrido[1,2-a]pyrazin-7-ylmethyl]-3*H*-benzoxazol-2-one;
(7R,9aS)-7-(4-fluorophenoxy)methyl-2-(5-fluoro-pyrimidin-2-yl )-2,3,4,6,7,8,9,9a-octahydro-1*H*-pyrido[1,2-a]pyrazine;
(7R,9aS)-7-(3,5-difluorophenoxy)methyl-2-(5-fluoro-pyrimidin-2-yl )-2,3,4,6,7,8,9,9a-octahydro-1 *H*-pyrido[1,2-a]pyrazine;
(7R,9aS)-7-(3,4-difluorophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(3-cyanophenoxy)methyl-2-(5-fluoro-pyrimidin-2-yl )-2,3,4,6,7,8,9,9a-octahydro-1 *H*-pyrido[1,2-a]pyrazine;
(7R,9aS)-7-(4-cyanophenoxy)methyl-2-(5-fluoro-pyrimidin-2-yl)-2,3,4,6,7,8,9,9a-octahydro-1 *H*-pyrido[1,2-a]pyrazine;
(7R,9aS)-7-(4-iodophenoxy)methyl-2-(5-fluoro-pyrimidin-2-yl )-2,3,4,6,7,8,9,9a-octahydro-1 *H*-pyrido[1,2-a]pyrazine;
(7R,9aS)-7-(4-fluorophenoxy)methyl-2-(4-fluorophenyl)-2,3,4,6,7,8,9,9a-octahydro-1*H*-pyrido[1,2-a]pyrazine;
(7S,9aS)-7-(4-fluorophenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(2-carbomethoxy-4-fluorophenoxy)methyl-2-(5-fluoro-pyrimidin-2-yl)-2,3,4,6,7,8,9,9a-octahydro-1 *H*-pyrido[1,2-a]pyrazine;
(75,9aS)-7-(2-bromo-4-fluorophenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(4-fluoro-2-trifluoromethylphenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(3,5-difluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluorophenoxy)methyl-2-(5-chloro-pyridin-2-yl)-2,3,4,6,7,8,9,9a-octahydro-1*H*-pyrido[1,2-a]pyrazine;
(7S,9aS)-7-(4-fluoro-2-methylphenoxy)methyl-2-(5-fluoro-pyrimidin-2-yl)-2,3,4,6,7,8,9,9a-octahydro-1 *H*-pyrido[1,2-a]pyrazine;
(7S,9aS)-7-(2,4-difluorophenoxy)methyl-2-(5-fluoro-pyrimidin-2-yl )-2,3,4,6,7,8,9,9a-octahydro-1*H*-pyrido[1,2-a]pyrazine;
(7S,9aS)-7-(3-methyl-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,4-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3,5-difluoro-phenoxy)methyl-2-(5-fluoropyrimidin-2-yl)-2,3,4,6,7,8,9,9a-octahydro-1*H*-pyrido[1,2-a]pyrazine;
(7S,9aS)-7-(3-cyano-phenoxy)methyl-2-(5-fluoropyrimidin-2-yl )-2,3,4,6,7,8,9,9a-octahydro-1 *H*-pyrido[1,2-a]pyrazine;
(7S,9aS)-7-(3-trifluoromethyl-phenoxy)methyl-2-(5-fluoropyrimidin-2-yl )-2,3,4,6,7,8,9,9a-octahydro-1*H*-pyrido[1,2-a]pyrazine;
(7S,9aS)-7-(4-trifluoromethyl-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-trifluoromethoxy-phenoxy)methyl-2-(5-fluoropyrimidin-2-yl)-2,3,4,6,7,8,9,9a-octahydro-1 *H*-pyrido[1,2-a]pyrazine;
(7S,9aS)-7-(3-methoxy-phenoxy)methyl-2-(5-fluoropyrimidin-2-yl)-2,3,4,6,7,8,9,9a-octahydro-1*H*-pyrido[1,2-a]pyrazine; and
(7S,9aS)-7-(4-methoxy-phenoxy)methyl-2-(5-fluoropyrimidin-2-yl )-2,3,4,6,7,8,9,9a-octahydro-1 *H*-pyrido[1,2-a]pyrazine;

15. A method according to Claim 10, wherein the D4 dopamine receptor antagonist that is employed in such a method is selected from the following compounds of their pharmaceutically acceptable salts:
(7S,8aS)-7-(4-fluorophenoxy)methyl-2-(5-chloropyridin-2-yl)-1,2,3,4,6,7,8,8a-octahydro-pyrrolo[1,2-a]pyrazine;
(7S,8aS)-7-(3,5-difluorophenoxy)methyl-2-(5-chloropyridin-2-yl )-1,2,3,4,6,7,8,8a-octahydro-pyrrolo[1,2-a]pyrazine;
(7S,8aS)-7-(3-cyanophenoxy)methyl-2-(5-chloropyidin-2-yl )-1,2,3,4,6,7,8,8a-octahydro-pyrrolo[1,2-a]pyrazine;
(7S,8aS)-7-(4-cyanophenoxy)methyl-2-(5-chloropyidin-2-yl )-1,2,3,4,6,7,8,8a-octahydro-pyrrolo[1,2-a]pyrazine;
(7S,8aS)-7-(4-fluorobenzyl)oxy-2-(5-chloropyridin-2-yl)-1,2,3,4,6,7,8,8a-octahydro-pyrrolo[1,2-a]pyrazine;
(7S,8aS)-2-(5-chloropyridin-2-yl)-1,2,3,4,6,7,8,8a-octahydro-pyrrolo[1,2-a]pyrazin-7-yl benzoate;
(7S,8aS)-7-(4-fluorophenoxy)methyl-2-(5-fluoropyrimidin-2-yl)-1,2,3,4,6,7,8,8a-octahydro-pyrrolo[1,2-a]pyrazine;
(7S,8aS)-7-(3,5-difluorophenoxy)methyl-2-(5-fluoropyrimidin-2-yl)-1,2,3,4,6,7,8,8a-octahydro-pyrrolo[1,2-a]pyrazine;
(7S,8aS)-7-(3-cyanophenoxy)methyl-2-(5-fluoropyrimidin-2-yl)-1,2,3,4,6,7,8,8a-octahydro-pyrrolo[1,2-a]pyrazine;
(7S,8aS)-7-(4-cyanophenoxy)methyl-2-(5-fluoropyrimidin-2-yl)-1,2,3,4,6,7,8,8a-octahydro-pyrrolo[1,2-a]pyrazine;
(7S,8aS)-7-(4-fluorobenzyl)oxy-2-(5-fluoropyrimidin-2-yl)-1,2,3,4,6,7,8,8a-octahydro-pyrrolo[1,2-a]pyrazine;
(7S,8aS)-2-(5-fluoropyrimidin-2-yl)-1,2,3,4,6,7,8,8a-octahydro-pyrrolo[1,2-a]pyrazin-7-yl benzoate; and
(7S,8aS)-7-(3-cyanobenzyl)oxy-2-(5-fluoropyrimidin-2-yl)-1,2,3,4,6,7,8,8a-octahydro-pyrrolo[1,2-a]pyrazine.

16. A method according to claim 10 when the D4 dopamine receptor antagonist that is employed in such a method is selected from the following compounds and their pharmaceutically acceptable salts
1- (2, 5 -dichlorophenyl) - 4 - (3, 4, 5 - trimethoxyhenzyl) -piperazine;
1- (2, 3 -dichlorophenyl) - 4 - (3, 4, 5 - trimethoxybenzyl) -piperazine;
1 - (3, 4 -dichlorophenyl) - 4 - (3, 4, 5 - trimethoxybenzyl) -piperazine;
1-(2,3-dimethylphenyl)-4-(3,4,5-trimethoxybenzyl)-piperazine;
1-(3, 4 -dimethylphenyl) - 4 - (3, 4, 5 - trimethoxybenzyl) - piperazine;
1 - (2 - chloro - 3 -methyphenyl) - 4 - (3, 4, 5 - trimethoxybenzyl) piperazine;
1 - (2 - chloro - 4 -methyphenyl) - 4 - (3, 4, 5 - trimethoxybenzyl) piperazine;
1-(2-chloro-5-methylphenyl)-4-(3,4,5-trimethoxybenzyl) piperazine;
1-(3 - chloro- 2 -methyphenyl) - 4 - (3, 4, 5 - trimethoxvbenzyl) -piperazine;
1- (3 - chloro- 4 -methyphenyl) - 4 - (3, 4, 5 - trimethoxvbenzyl) -piperazine;
1- (5 - chloro - 2 -methyphenyl) - 4-; (3,4,5- trimethoxvbenzyl) -piperazine
1- (3-chloro-4-methylphenyl) -4- (3, 4, 5-trimethoxy-benzyl) piperazine;
1-(5-chloro-2-methylphenyl)-4-(3,4,5-trimethoxy-benzyl) piperazine;
1- (4 -chloro - 2 -methyphenyl) - 4 - (3, 4, 5 - trimethoxy -benzyl) piperazine;
1- (4 - chloro-3 -me thylphenyl) - 4 - (3, 4, 5 - trimethoxy -benzyl) piperazine;
1-pyridin-2-yl-4-(3,4,5-trimethoxybenzyl)-piperazine; and
4-phenyl-1-(3,4,5-trimethoxybenzyl)piperidine;
1-phenyl-4- (3 4, 5-trrimethoxybenzyl) piperazine;
1-(2-chlorophenyl)-4-(3,4,5-trimethoxybenzyl)-piperazine;
1-(3-chlorophenyl)-4-(3,4,5-trimethoxybenzyl)-piperazine;
1-(4-chlorophenyl)-4-(3,4,5- trimethoxybenzyl)-piperazine;
1-o-tolyl-4- (3, 4, 5-trimethoxybenzyl) piperazine;
1-m-tolyl-4-(3,4,5-trimethoxybenzyl)piperazine;
1-m-tolyl-4-(3,4,5-trimethoxybenzyl)piperazine;
1-(2-methoxyphenyl)-4-(3,4,5-trimethoxybenzyl)-piperazine;
1-(3-methoxyphenyl)-4-(3,4,5-trimethoxybenzyl)-piperazine;
1-(4-methoxyphenyl)-4-(3,4,5-trimethoxybenzyl)-piperazine;
1-(2,5-dichlorophenyl)-4-(3,4,5-trimehoxybenzyl)-piperazine;
1-(2,3-dichlorophenyl)-4-(1,4,5-trimethoxybenzyl)-piperazine;
1-(3,4-dichlorophenyl)-4-(3,4,5-trimethoxybenzyl)-piperazine;
1-(2,3-dimethylphenyl)-4-(34,5-trimethoxybenzyl)-piperazine;
1-(3,4-dimethylphenyl )-4-(3,4,5-trimethoxybenzyl)-piperazine;
1-(2-chloro-3-methylphenyl)-4-~3,4,5-trimethoxybenzyl) piperazine;
1- (2-chloro-4-methylphenyl) -4- (3, 4, 5-trimethoxybenzyl) piperazine;
1-(2-chloro-5-methylphenyl)-4-(3,4,5-trimethoxy) benzyl) piperazine;
1- (3-chloro-2-methylphenyl) -4- (3, 4, 5-trimethoxybenzyl) piperazine;
1- (3-chloro-4-methylphenyl) -4- (3, 4, 5-trimethoxybenzyl) piperazine;
1-(5-chloro-2-methylphenyl)-4-(3,4,5-trime~hoxy-benzyl) piperazine;
1-(4-chloro-2-meLhylphenyl)-4-(3,4,5-trimethoxy-benzyl) piperazine;
1-(4-chloro-3-methylohenyl)-4-(3,4,5-trimethoxy benzyl) piperazine;
1-pyridin-2-yl-4- (3,4,5-trimethoxybenzyl)-piperazine; and
4-phenyl-1- (3,4,5-trimethoxybenzyl) piperidine;
1- (2 -chloro- 3 -methyphenyl) - 4 - (2, 3 -dimethoxybenzyl) piperazine;
1-(2-chloro-3-methylphenyl)-4-(2,4-dimethoxybenzyl) piperazine;
1-(2-chloro-3-methylphenyl)-4-(2,5-dimethoxybenzyl) piperazine; and
1-(2-chloro-3 -methyphenyl)-4-(3, 4-dimethoxybenzyl) piperazine;
1- (2-chloro-3-methylphenyl) -4- (2, 3-dimethoxybenzyl) piperazine;
1- (2-chloro-3-methylphenyl) -4- (2, 4-dimethoxybenzyl) piperazine;
1 (2-chloro-3-methylphenyl)-4-(2,5-dimethoxybenzyl) piperazine; and
1-(2-chloro-3-methylphenyl)-4-(3,4-dimethoxybenzyl), piperazine;

17. The pharmaceutical composition according to Claim 1 wherein the D4 dopamine receptor antagonist that is employed in such a method is selected from the following compounds and their pharmaceutically acceptable salts:
4-(4-(3-oxo-3,4-dihydro-2H-benzo[1,4]oxazin-6-ylmethyl)-piperazin-1-yl]-benxenesulfonamide;
6-[4-(3, 4-dimethyl-phenyl)-piperazin-1-ylmethyl]-4H-benzo[1,4]oxazin-3-one;
6-(4-p-tolyl-piperazin-1-ylmethyl)-4H-benzo[1,4]oxazin-3-one;
6-[4-phenyl-piperazin-1 -ylmethyl]-4H-benzo[1,4]oxazin-3-one;
7-(4-p-tolyl-piperazin-I-ylmethyl-4H-benzo[1,41oxazin-3-one;
7-(4-phenyl-piperazin-I-ylmethyl)-4H-benzo[1,4]oxazine-3-one;
7-[4-(3,4-dimethyl-phenyl)-piperazin-1-ylmethyl)-4H-benzo[1,4]oxazine-3-one;
6-[4-(5-methyl-pyrrdin-2-yl)-piperazin-1-ylmethyl]-4H-benzo[1,4]oxazin-3-one;
6-(4-p-tolyl-piperidin-1-ylmethyl)-4H-benxo1,4]oxazin-3-one;
6-[4-(3,4-Dimethyl-phenyl)piperidin-1-ylmethyl]-4H-benzo[1,4]oxazin-3-one;
6-(4-thiazol-2-yl-piperazin-1-ylmethyl)-4H-benzo(1,4]oxazin-3-one;
6-(4-benzothiazol-2-yl-piperazin-1-ylmethyl)-4H-benzo[1,4]oxazin.-3-one;
6-(4-(4,5-dimethyl-thiazol-2-yl)-piperazin-1-ylmethyl]-4H-benzo[1,4]oxazin-3-one;
6-(4-naphthalen-2-yl-piperazin-1-ylmethyl)-4H-benzo[1,4]oxazin-3-one;
6-[4-(3-chloro-phenyl)-piperazin-1-ylmethyl]-4H- benzo[1,4]oxazin-3-one;
6-[4-(3,4-dichloro-phenyl)-piperaziin-1-ylmethyl)-4H-benzo[1,4]oxazin-3-one;
2-[4-(3-oxo-3,4-dihydro-2H-benzo[1,4]oxazin-6-ylmethyl)-piperazin-1-yl]-benzonitrile;
6-[4-(4-methoxy-phenyl)-piparazin-1-ylmethyl]-4H-benzo[1,4]oxazin-3-one;
6-[4-(2-chloro-4-methyl-phenyl )-piperazin-1-ylmethyl]-4H-benzo[1,4]oxazin-3-one;
6-(4-(4-Fluoro)-phenyl)-piperazin-1-ylmethyl]-4H-benzo[1,4]oxazin-3-one;
6-[4-(3-Trifluoromethyl-phenyl)-piperazin-1-ylmethyl]-4H-benzo[1,4]oxazin-3-one;
6-[4-(3,5-Dimethyl-phenyl)-piperaazin-1-ylmethyl]-4H-benzo[1,4]oxazin-3-one;
6-[4-(2-Chloro-phenyl)-piperazin-1-ylmethyl]-4H-benzo[1,4]oxazin-3-one;
6-[4-(4-Trifluoromethyl-phenyl)-piperazin-1-ylmethyl]-4H-benzo[1,4]oxazin-3-one;
6-[4-(4-Chloro-phenyl)-piperazin-1-ylmethyl]-4H-benzo[1,4]oxazin-3-one;
7-[4-(5-Methyl-pyridin-2-yl)-piperazin-1-ylmethyl]-4H-benzo[1,4]oxazin-3-one;
7-[4-(4-Methoxy-phenyl)-piperazin-1-yimethyl]-4H-benzo[1,4]oxazin-3-one,
7-[4-(4-Chloro-phenyl)-piperazin-1-ylmethyl]-4H-benzo[1,4]oxazin-3-one;
7-[4-(3,4-Dimethyl-phenyl)-piperidin-1-ylmethy]-4H-benzo[1,4]oxazin-3-one;
6-[4-(4-Methoxy-phenyl)-piperidin-1-ylmethyl]-4H-benzo(1,4]oxazin-3-one;
7-[4-(4-Methoxy-phenyl)-piperidin-1-ylmethyl]-4H-benzo(1,4]oxazin-3-one;
7-(4-Phenyl-piperidin-1-ylmethyl)-4H-benzo[1,4]oxazin-3-one;
7-(4-Naphthalen-2-yl-piperazin-1-ylmethy)-4H-benzo[1,4)oxazin-3-one; and
7-(4-p-Tolyl-piperidin-1-ylmethyl)-4H-benzo[1,4)oxazin-3-one.
